# EUROPÄISCHE PATENTANMELDUNG

(11) **EP 1 172 375 A1**
(43) Veröffentlichungstag der Anmeldung: **16.01.2002**
(21) Anmeldenummer: 00128446.2
(22) Anmeldetag: 22.12.2000
(51) Int. Cl.: C07K 14/47

(54) **Genexpression, Genomalteration und Reporterexpression in Myofibroblasten und Myofibroblast-ähnlichen Zellen durch Verwendung der regulatorischen Bereiche des Alpha Smooth Muscle Aktin-Gens**

(30) Priorität: 11.07.2000 DE 10033633; 31.10.2000 DE 10053879
(71) Anmelder: Odenthal, Margarete, 50937 Köln (DE)
(72) Erfinder: Odenthal, Margarete, Dr., 50937 Köln (DE); Jung, Diana, 8003 Zürich (CH)
(74) Vertreter: Helbing, Jörg, Dr. Dipl.-Chem.

(57) **Zusammenfassung**

Die Erfindung betrifft spezifische, regulatorische Sequenzbereiche des alpha Smooth Muscle Aktin-Gens (α-SMA-Gens), Fusionskonstrukte, in denen solche regulatorische Sequenzbereiche des α-SMA-Gens mit weiteren funktionellen Nukleinsäuresequenzen operativ verknüpft sind, sowie die Verwendung solcher regulatorischer Sequenzbereiche zur zelltyp- und differenzierungspezifischen Reporterexpression, zu zelltyp- und differenzierungsspezifischen Genveränderungen (Alteration, Mutation) und zu Genexpressionsveränderungen in Zellen und Organismen.

SEQUENZPROTOKOLL

<110> Odenthal, Margarete

<120> Genexpression, Genomalteration und Reporterexpression
      in Myofibroblasten und Myofibroblasten-ähnlichen Zellen

<130> 002086ep/JH/ml

<140>
<141>

<160> 41

<170> PatentIn Ver. 2.1

<210> 1
<211> 764
<212> DNA
<213> Rattus norvegicus

<400> 1
acggtcctta agcatgatat caagggtcag cgataaacca acaacatgca cgtggactgt 60
acctaagggt taacgcagtt acagtgattc tgacttctaa gttcctctta gggtaacata 120
ggctggtgaa tcctgattac atacttccat atgtaataca tacagacttc attgatacta 180
cacacagact ccagactaca tacaatgtgg cttccataaa atgatcactc ctctgcagat 240
tcgcaggtga cccaagcatc ttttgttata ggctaccttt tgcaacagtg ttgccttaaa 300
gtcccagcta gtcagagaca ggcccttcct catctcaagc ccttagctaa tggacccaaa 360
ggctagcctg acaggaagag ctggcatctt ctgaggaatg tgcaaaccat gcctgcgtct 420
gcttcatgac actagcccag tgtctgggca tttgagcagt tgttctgagg gctcaggatg 480
tttatcccca taagcagctg aactgcctcc tgtttcgaga gcagagcaga ggaatgcagt 540
ggaagagacc cacgctctgg ccacccagat tagagagttt tgtgctgagg tccctatatg 600
gttgtgttag agtgaacggc cagcttcagc ctgtctttgc tccttgtttg ggaagcgagt 660
gggaggggat cagaccaggg ggctatataa cccttcagca ttcagcctcc ccagacacca 720
cccacccaga gtggagaagc ccagccagtc gccatcaggg taag 764


<210> 2
<211> 745
<212> DNA
<213> Mus musculus

<400> 2
atggtcctta atcatgctgt taagggtcag taaaaagcca gcaacatgct ggaatgttaa 60
gggttaaagc agttacagtg attctgactt ctaagttact ctttgggcaa cacaggctgg 120
ttaatcctca ctacatactt caattcctat ttccactcac ctccctcaag aacttgattt 180
ataaacagtg tgcctaccat aaaatcatga ctcctctgca tatttatggg tgactcgaag 240
catcttttga tctaggctac cttttgcaac agtgttgctt aaaaatcgca gctagtcaga 300
gacaggccct tccttatcca agtcctcagc taatggccca aaagactagc ctgacagggg 360
ctggcatctt ctgaggaatg tgcaaaccgt gcctgcgtct gtcccatgac actagcccag 420
tgtctgggca tttaagcagt tgttctgagg gcttaggatg tttatcccca taacgagctg 480
agctgcctcc tgtttcggga gcagaacaga ggaatgcagt ggaagagacc cacgctctgg 540
ccacccagat tagagagttt tgtgctgagg tccctatatg gttgtgttag agtgaacggc 600
cagcttcagc ccgtctttgc tccttgtttg ggaggcgagt gggaggggat cagagcaagg 660
ggctatataa cccttcagcc ttcagcctcc ccgggacacc acccacccag agtggagaag 720
cccagccagt cgctgtcagg gtaag 745

<210> 3
<211> 763
<212> DNA
<213> Homo sapiens

<400> 3
atggtcccta cttatgctgc taaattgctc ggtgacaaat tagtagacaa agctaatgca 60
caaaaaaatg aatgtagtta tagtaatgca gaattccaaa ttcctctttg taagacatag 120
gcctgtcaac cttgtctcca tacttcagtt cctggtttca ttgaaccaac acaaagacac 180
aatgtataag tacaatgtag cttccataaa aacatcactc cctctatgta tttatagaca 240
gctgaagaaa tatctttctt ctttgcatcc taccgtggta gaagggtttt aaaagtccgt 300
gctaggcaga ggcagccctt tctgcccctt tctgttctca gtttattagg aaatggcctg 360
aaattccagc atgatagcaa gctggcatcc tctgtggaat gtcaatacca tgcctgcatc 420
tgcccattac cctagctcag tgtctctggg catttctgca gttgttctga aggcttggcg 480
tgtttatctc ccacaagcgg ctgaactgcc tcccgtttca tgagcagacc agtggaatgc 540
agtggaagag acccacgctc cggccaccca gattagagag ttttgtgctg aggtccctat 600
atggttgtgt tagactgaac gacaggctca agtctgtctt tgctccttgt ttgggaagca 660
agtgggagga gagcaggcca aggggctata taacccttca gctttcagct tccctgaaca 720
ccacccagtg tggagcagcc cagccaagca ctgtcagggt aag 763

<210> 4
<211> 747
<212> DNA
<213> Gallus gallus

<400> 4
acactaggat gaagcttgtc cacagttcct agtgctttgg aaataaactg atggagacag 60
gatattgatt gtcacccatt acaggctagg gacaccataa caacctgtta gcagaacgtt 120
tacacagcct tcaaagaccc taccatgaac cctatgcaac agcaggtact tcttttagta 180
tccccccagt gcagaccttt taagtgaatt tgtggcaaaa ttcagtagct gtttagcttg 240
ccgaaagtat tctcattgct ttggtccaaa tctttaacta atgcaaagtg tctccttaaa 300
aacactttcc ctattacaaa tgactgctct ttcagttttc actctgcctc ttggatgttc 360
ctgtgaaggc cagggcctct ctctcttgtt tgaacgtgtg ctcttcctga cagagggtgt 420
ctgtcccagg cacgcttttc ttgctgcatt ttagcaagtt ctgcagtgtt tatcttacac 480
agctgaaagt ctcctcctgt ttcatgagct ctgcgttgga atgcagtgga agggactgac 540
gcctgtcgac ccagattaga ggtttttgta ataaggtccc tatatggttt tgttagagac 600
ttcggctctg tctctctcat ctctgctcct tgtttgggag gctggtggga ggagaagagc 660
tgaaggggct atataaccct ggtgcttttg gatacacagt gcaccatccc agagctgcta 720
tccccagcca agcactgtca gggtaag 747

<210> 5
<211> 716
<212> DNA
<213> Rattus norvegicus

<400> 5
tgatatcaag ggtcagcgat aaaccaacaa catgcacgtg gactgtacct aagggttaac 60
gcagttacag tgattctgac ttctaagttc ctcttagggt aacataggct ggtgaatcct 120
gattacatac ttccatatgt aatacataca gacttcattg atactacaca cagactccag 180
actacataca atgtggcttc cataaaatga tcactcctct gcagattcgc aggtgaccca 240
agcatctttt gttataggct accttttgca acagtgttgc cttaaagtcc cagctagtca 300
gagacaggcc cttcctcatc tcaagccctt agctaatgga cccaaaggct agcctgacag 360
gaagagctgg catcttctga ggaatgtgca aaccatgcct gcgtctgctc catggcacta 420
gcccagtgtc tgggcatttg agcagttgtt ctgagggctc aggatgttta tccccataag 480
cagctgaact gcctcctgtt tcgagagcag agcagaggaa tgcagtggaa gagacccagg 540
cctctggcac ccagattaga gagttttgtg ctgaggtccc tatatggttg tgttagagtg 600
aacggccagc ttcagcctgt ctttgctcct tgtttgggaa gcgagtggga ggggatcaga 660

ccagggggct atataaccct tcagcattca gcctccccag acaccaccca cccaga 716

<210> 6
<211> 142
<212> DNA
<213> Rattus norvegicus

<400> 6
ggtccctata tggttgtgtt agagtgaacg gccagcttca gcctgtcttt gctccttgtt 60
tgggaagcga gtgggagggg atcagaccag ggggctatat aacccttcag cattcagcct 120
ccccagacac cacccaccca ga 142

<210> 7
<211> 207
<212> DNA
<213> Rattus norvegicus

<400> 7
gagcagagga atgcagtgga agagacccag gcctctggca cccagattag agagttttgt 60
gctgaggtcc ctatatggtt gtgttagagt gaacggccag cttcagcctg tctttgctcc 120
ttgtttggga agcgagtggg aggggatcag accagggggc tatataaccc ttcagcattc 180
agcctcccca gacaccaccc acccaga 207

<210> 8
<211> 232
<212> DNA
<213> Rattus norvegicus

<400> 8
tgaactgcct cctgtttcga gagcagagca gaggaatgca gtggaagaga cccaggcctc 60
tggcacccag attagagagt tttgtgctga ggtccctata tggttgtgtt agagtgaacg 120
gccagcttca gcctgtcttt gctccttgtt tgggaagcga gtgggagggg atcagaccag 180
ggggctatat aacccttcag cattcagcct ccccagacac cacccaccca ga 232

<210> 9
<211> 262
<212> DNA
<213> Rattus norvegicus

<400> 9
gggctcagga tgtttatccc cataagcagc tgaactgcct cctgtttcga gagcagagca 60
gaggaatgca gtggaagaga cccaggcctc tggcacccag attagagagt tttgtgctga 120
ggtccctata tggttgtgtt agagtgaacg gccagcttca gcctgtcttt gctccttgtt 180
tgggaagcga gtgggagggg atcagaccag ggggctatat aacccttcag cattcagcct 240
ccccagacac cacccaccca ga 262

<210> 10
<211> 502
<212> DNA
<213> Rattus norvegicus

<400> 10
tcctctgcag attcgcaggt gacccaagca tcttttgtta taggctacct tttgcaacag 60
tgttgcctta aagtcccagc tagtcagaga caggcccttc ctcatctcaa gcccttagct 120

aatggaccca aaggctagcc tgacaggaag agctggcatc ttctgaggaa tgtgcaaacc 180
atgcctgcgt ctgctccatg gcactagccc agtgtctggg catttgagca gttgttctga 240
gggctcagga tgtttatccc cataagcagc tgaactgcct cctgtttcga gagcagagca 300
gaggaatgca gtggaagaga cccaggcctc tggcacccag attagagagt tttgtgctga 360
ggtccctata tggttgtgtt agagtgaacg gccagcttca gcctgtcttt gctccttgtt 420
tgggaagcga gtgggagggg atcagaccag ggggctatat aacccttcag cattcagcct 480
ccccagacac cacccaccca ga 502

<210> 11
<211> 539
<212> DNA
<213> Rattus norvegicus

<400> 11
cagactacat acaatgtggc ttccataaaa tgatcactcc tctgcagatt cgcaggtgac 60
ccaagcatct tttgttatag gctacctttt gcaacagtgt tgccttaaag tcccagctag 120
tcagagacag gcccttcctc atctcaagcc cttagctaat ggacccaaag gctagcctga 180
caggaagagc tggcatcttc tgaggaatgt gcaaaccatg cctgcgtctg ctccatggca 240
ctagcccagt gtctgggcat ttgagcagtt gttctgaggg ctcaggatgt ttatccccat 300
aagcagctga actgcctcct gtttcgagag cagagcagag gaatgcagtg gaagagaccc 360
aggcctctgg cacccagatt agagagtttt gtgctgaggt ccctatatgg ttgtgttaga 420
gtgaacggcc agcttcagcc tgtctttgct ccttgtttgg gaagcgagtg ggaggggatc 480
agaccagggg gctatataac ccttcagcat tcagcctccc cagacaccac ccacccaga 539

<210> 12
<211> 65
<212> DNA
<213> Rattus norvegicus

<400> 12
gagcagagga atgcagtgga agagacccag gcctctggca cccagattag agagttttgt 60
gctga 65

<210> 13
<211> 25
<212> DNA
<213> Rattus norvegicus

<400> 13
tgaactgcct cctgtttcga gagca 25

<210> 14
<211> 30
<212> DNA
<213> Rattus norvegicus

<400> 14
gggctcagga tgtttatccc cataagcagc 30

<210> 15
<211> 240
<212> DNA
<213> Rattus norvegicus

<400> 15
tcctctgcag attcgcaggt gacccaagca tcttttgtta taggctacct tttgcaacag 60
tgttgcctta aagtcccagc tagtcagaga caggcccttc ctcatctcaa gcccttagct 120
aatggaccca aaggctagcc tgacaggaag agctggcatc ttctgaggaa tgtgcaaacc 180
atgcctgcgt ctgctccatg gcactagccc agtgtctggg catttgagca gttgttctga 240

<210> 16
<211> 37
<212> DNA
<213> Rattus norvegicus

<400> 16
cagactacat acaatgtggc ttccataaaa tgatcac 37

<210> 17
<211> 177
<212> DNA
<213> Rattus norvegicus

<400> 17
tgatatcaag ggtcagcgat aaaccaacaa catgcacgtg gactgtacct aagggttaac 60
gcagttacag tgattctgac ttctaagttc ctcttagggt aacataggct ggtgaatcct 120
gattacatac ttccatatgt aatacataca gacttcattg atactacaca cagactc 177

<210> 18
<211> 697
<212> DNA
<213> Mus musculus

<400> 18
tgctgttaag ggtcagtaaa aagccagcaa catgctggaa tgttaagggt taaagcagtt 60
acagtgattc tgacttctaa gttactcttt gggcaacaca ggctggttaa tcctcactac 120
atacttcaat tcctatttcc actcacctcc ctcaagaact tgatttataa acagtgtgcc 180
taccataaaa tcatgactcc tctgcatatt tatgggtgac tcgaagcatc ttttgatcta 240
ggctaccttt tgcaacagtg ttgcttaaaa atcgcagcta gtcagagaca ggcccttcct 300
tatccaagtc ctcagctaat ggcccaaaag actagcctga caggggctgg catcttctga 360
ggaatgtgca aaccgtgcct gcgtctgtcc catgacacta gcccagtgtc tgggcattta 420
agcagttgtt ctgagggctt aggatgttta tccccataac gagctgagct gcctcctgtt 480
tcgggagcag aacagaggaa tgcagtggaa gagacccacg ctctggccac ccagattaga 540
gagttttgtg ctgaggtccc tatatggttg tgttagagtg aacggccagc ttcagcccgt 600
ctttgctcct tgtttgggag gcgagtggga ggggatcaga gcaaggggct atataaccct 660
tcagccttca gcctccccgg gacaccaccc acccaga 697

<210> 19
<211> 715
<212> DNA
<213> Homo sapiens

<400> 19
tgctgctaaa ttgctcggtg acaaattagt agacaaagct aatgcacaaa aaaatgaatg 60
tagttatagt aatgcagaat tccaaattcc tctttgtaag acataggcct gtcaaccttg 120
tctccatact tcagttcctg gtttcattga accaacacaa agacacaatg tataagtaca 180
atgtagcttc cataaaaaca tcactccctc tatgtattta tagacagctg aagaaatatc 240
tttcttcttt gcatcctacc gtggtagaag ggttttaaaa gtccgtgcta ggcagaggca 300
gccctttctg cccctttctg ttctcagttt attaggaaat ggcctgaaat tccagcatga 360

tagcaagctg gcatcctctg tggaatgtca ataccatgcc tgcatctgcc cattacccta 420
gctcagtgtc tctgggcatt tctgcagttg ttctgaaggc ttggcgtgtt tatctcccac 480
aagcggctga actgcctccc gtttcatgag cagaccagtg gaatgcagtg gaagagaccc 540
acgctccggc cacccagatt agagagtttt gtgctgaggt ccctatatgg ttgtgttaga 600
ctgaacgaca ggctcaagtc tgtctttgct ccttgtttgg gaagcaagtg ggaggagagc 660
aggccaaggg gctatataac ccttcagctt tcagcttccc tgaacaccac ccagt 715

<210> 20
<211> 699
<212> DNA
<213> Gallus gallus

<400> 20
cttgtccaca gttcctagtg ctttggaaat aaactgatgg agacaggata ttgattgtca 60
cccattacag gctagggaca ccataacaac ctgttagcag aacgtttaca cagccttcaa 120
agaccctacc atgaacccta tgcaacagca ggtacttctt ttagtatccc cccagtgcag 180
accttttaag tgaatttgtg gcaaaattca gtagctgttt agcttgccga aagtattctc 240
attgctttgg tccaaatctt taactaatgc aaagtgtctc cttaaaaaca ctttccctat 300
tacaaatgac tgctctttca gttttcactc tgcctcttgg atgttcctgt gaaggccagg 360
gcctctctct cttgtttgaa cgtgtgctct tcctgacaga gggtgtctgt cccaggcacg 420
cttttcttgc tgcattttag caagttctgc agtgtttatc ttacacagct gaaagtctcc 480
tcctgtttca tgagctctgc gttggaatgc agtggaaggg actgacgcct gtcgacccag 540
attagaggtt tttgtaataa ggtccctata tggttttgtt agagacttcg gctctgtctc 600
tctcatctct gctccttgtt tgggaggctg gtgggaggag aagagctgaa ggggctatat 660
aaccctggtg cttttggata cacagtgcac catcccaga 699

<210> 21
<211> 845
<212> DNA
<213> Künstliche Sequenz

<220>
<223> Beschreibung der künstlichen Sequenz: EGFP

<400> 21
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgaccta cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagaag 720
cttagccatg gcttcccgcc ggaggtggag gagcaggatg atggcacgct gcccatgtct 780
tgtgcccagg agagcgggat ggaccgtcac cctgcagcct gtgcttctgc taggatcaat 840
gtgta 845

<210> 22
<211> 666
<212> DNA
<213> Künstliche Sequenz

<220>
<223> Beschreibung der künstlichen Sequenz: Red
      Fluorescent Protein

<400> 22
aagaatgtta tcaaggagtt catgaggttt aaggttcgca tggaaggaac ggtcaatggg 60
cacgagtttg aaatagaagg cgaaggagag gggaggccat acgaaggcca caataccgta 120
aagcttaagg taaccaaggg gggacctttg ccatttgctt gggatatttt gtcaccacaa 180
tttcagtatg gaagcaaggt atatgtcaag caccctgccg acataccaga ctataaaaag 240
ctgtcatttc ctgaaggatt taaatgggaa agggtcatga actttgaaga cggtggcgtc 300
gttactgtaa cccaggattc cagtttgcag gatggctgtt tcatctacaa ggtcaagttc 360
attggcgtga actttccttc cgatggacct gttatgcaaa agaagacaat gggctgggaa 420
gccagcactg agcgtttgta tcctcgtgat ggcgtgttga aaggagagat tcataaggct 480
ctgaagctga aagacggtgg tcattaccta gttgaattca aaagtattta catggcaaag 540
aagcctgtgc agctaccagg gtactactat gttgactcca aactggatat aacaagccac 600
aacgaagact atacaatcgt tgagcagtat gaaagaaccg agggacgcca ccatctgttc 660
ctttag                                                            666


<210> 23
<211> 719
<212> DNA
<213> Künstliche Sequenz

<220>
<223> Beschreibung der künstlichen Sequenz: GFP

<400> 23
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgaccca cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaacttcaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagta  719


<210> 24
<211> 937
<212> DNA
<213> Künstliche Sequenz

<220>
<223> Beschreibung der künstlichen Sequenz: Renilla
      Luciferase

<400> 24
atgacttcga aagtttatga tccagaacaa aggaaacgga tgataactgg tccgcagtgg 60
tgggccagat gtaaacaaat gaatgttctt gattcattta ttaattatta tgattcagaa 120
aaacatgcag aaaatgctgt tattttttta catggtaacg cggcctcttc ttatttatgg 180
cgacatgttg tgccacatat tgagccagta gcgcggtgta ttataccaga ccttattggt 240
atgggcaaat caggcaaatc tggtaatggt tcttataggt tacttgatca ttacaaatat 300
cttactgcat ggtttgaact tcttaattta ccaaagaaga tcatttttgt cggccatgat 360
tggggtgctt gtttggcatt tcattatagc tatgagcatc aagataagat caaagcaata 420

gttcacgctg aaagtgtagt agatgtgatt gaatcatggg atgaatggcc tgatattgaa 480
gaagatattg cgttgatcaa atctgaagaa ggagaaaaaa tggttttgga gaataacttc 540
ttcgtggaaa ccatgttgcc atcaaaaatc atgagaaagt tagaaccaga agaatttgca 600
gcatatcttg aaccattcaa agagaaaggt gaagttcgtc gtccaacatt atcatggcct 660
cgtgaaatcc cgttagtaaa aggtggtaaa cctgacgttg tacaaattgt taggaattat 720
aatgcttatc tacgtgcaag tgatgattta ccaaaaatgt ttattgaatc ggacccagga 780
ttcttttcca atgctattgt tgaaggtgcc aagaagtttc ctaatactga atttgtcaaa 840
gtaaaaggtc ttcatttttc gcaagaagat gcacctgatg aaatgggaaa atatatcaaa 900
tcgttcgttg agcgagttct caaaaatgaa caataat 937

<210> 25
<211> 3144
<212> DNA
<213> Künstliche Sequenz

<220>
<223> Beschreibung der künstlichen Sequenz: beta
      Galactosidase

<400> 25
atgtcgttta ctttgaccaa caagaacgtg attttcgttg ccggtctggg aggcattggt 60
ctggacacca gcaaggagct gctcaagcgc gatcccgtcg ttttacaacg tcgtgactgg 120
gaaaaccctg gcgttaccca acttaatcgc cttgcagcac atcccccttt cgccagctgg 180
cgtaatagcg aagaggcccg caccgatcgc ccttcccaac agttgcgcag cctgaatggc 240
gaatggcgct ttgcctggtt tccggcacca gaagcggtgc cggaaagctg gctggagtgc 300
gatcttcctg aggccgatac tgtcgtcgtc ccctcaaact ggcagatgca cggttacgat 360
gcgcccatct acaccaacgt aacctatccc attacggtca atccgccgtt tgttcccacg 420
gagaatccga cgggttgtta ctcgctcaca tttaatgttg atgaaagctg gctacaggaa 480
ggccagacgc gaattatttt tgatggcgtt aactcggcgt ttcatctgtg gtgcaacggg 540
cgctgggtcg gttacggcca ggacagtcgt ttgccgtctg aatttgacct gagcgcattt 600
ttacgcgccg gagaaaaccg cctcgcggtg atggtgctgc gttggagtga cggcagttat 660
ctggaagatc aggatatgtg gcggatgagc ggcattttcc gtgacgtctc gttgctgcat 720
aaaccgacta cacaaatcag cgatttccat gttgccactc gctttaatga tgatttcagc 780
cgcgctgtac tggaggctga agttcagatg tgcggcgagt tgcgtgacta cctacgggta 840
acagtttctt tatggcaggg tgaaacgcag gtcgccagcg gcaccgcgcc tttcggcggt 900
gaaattatcg atgagcgtgg tggttatgcc gatcgcgtca cactacgtct gaacgtcgaa 960
aacccgaaac tgtggagcgc cgaaatcccg aatctctatc gtgcggtggt tgaactgcac 1020
accgccgacg gcacgctgat tgaagcagaa gcctgcgatg tcggtttccg cgaggtgcgg 1080
attgaaaatg gtctgctgct gctgaacggc aagccgttgc tgattcgagg cgttaaccgt 1140
cacgagcatc atcctctgca tggtcaggtc atggatgagc agacgatggt gcaggatatc 1200
ctgctgatga agcagaacaa ctttaacgcc gtgcgctgtt cgcattatcc gaaccatccg 1260
ctgtggtaca cgctgtgcga ccgctacggc ctgtatgtgg tggatgaagc caatattgaa 1320
acccacggca tggtgccaat gaatcgtctg accgatgatc cgcgctggct accggcgatg 1380
agcgaacgcg taacgcgaat ggtgcagcgc gatcgtaatc acccgagtgt gatcatctgg 1440
tcgctgggga atgaatcagg ccacggcgct aatcacgacg cgctgtatcg ctggatcaaa 1500
tctgtcgatc cttcccgccc ggtgcagtat gaaggcggcg gagccgacac cacggccacc 1560
gatattattt gcccgatgta cgcgcgcgtg gatgaagacc agcccttccc ggctgtgccg 1620
aaatggtcca tcaaaaaatg gctttcgcta cctggagaga cgcgcccgct gatcctttgc 1680
gaatacgccc acgcgatggg taacagtctt ggcggtttcg ctaaatactg gcaggcgttt 1740
cgtcagtatc cccgtttaca gggcggcttc gtctgggact gggtggatca gtcgctgatt 1800
aaatatgatg aaaacggcaa cccgtggtcg gcttacggcg gtgattttgg cgatacgccg 1860
aacgatcgcc agttctgtat gaacggtctg gtctttgccg accgcacgcc gcatccagcg 1920
ctgacggaag caaaacacca gcagcagttt ttccagttcc gtttatccgg gcaaaccatc 1980
gaagtgacca gcgaatacct gttccgtcat agcgataacg agctcctgca ctggatggtg 2040
gcgctggatg gtaagccgct ggcaagcggt gaagtgcctc tggatgtcgc tccacaaggt 2100
aaacagttga ttgaactgcc tgaactaccg cagccggaga gcgccgggca actctggctc 2160
acagtacgcg tagtgcaacc gaacgcgacc gcatggtcag aagccgggca catcagcgcc 2220
tggcagcagt ggcgtctggc ggaaaacctc agtgtgacgc tccccgccgc gtcccacgcc 2280

atcccgcatc tgaccaccag cgaaatggat ttttgcatcg agctgggtaa taagcgttgg 2340
caatttaacc gccagtcagg ctttctttca cagatgtgga ttggcgataa aaaacaactg 2400
ctgacgccgc tgcgcgatca gttcacccgt gcaccgctgg ataacgacat tggcgtaagt 2460
gaagcgaccc gcattgaccc taacgcctgg gtcgaacgct ggaaggcggc gggccattac 2520
caggccgaag cagcgttgtt gcagtgcacg gcagatacac ttgctgatgc ggtgctgatt 2580
acgaccgctc acgcgtggca gcatcagggg aaaaccttat ttatcagccg gaaaacctac 2640
cggattgatg gtagtggtca aatggcgatt accgttgatg ttgaagtggc gagcgataca 2700
ccgcatccgg cgcggattgg cctgaactgc cagctggcgc aggtagcaga gcgggtaaac 2760
tggctcggat tagggccgca agaaaactat cccgaccgcc ttactgccgc ctgttttgac 2820
cgctgggatc tgccattgtc agacatgtat accccgtacg tcttcccgag cgaaaacggt 2880
ctgcgctgcg ggacgcgcga attgaattat ggcccacacc agtggcgcgg cgacttccag 2940
ttcaacatca gccgctacag tcaacagcaa ctgatggaaa ccagccatcg ccatctgctg 3000
cacgcggaag aaggcacatg gctgaatatc gacggtttcc atatggggat tggtggcgac 3060
gactcctgga gcccgtcagt atcggcggaa ttacagctga gcgccggtcg ctaccattac 3120
cagttggtct ggtgtcaaaa ataa 3144

<210> 26
<211> 1558
<212> DNA
<213> Künstliche Sequenz

<220>
<223> Beschreibung der künstlichen Sequenz: SEAP

<400> 26
atgctgctgc tgctgctgct gctgggcctg aggctacagc tctccctggg catcatccca 60
gttgaggagg agaacccgga cttctggaac cgcgaggcag ccgaggccct gggtgccgcc 120
aagaagctgc agcctgcaca gacagccgcc aagaacctca tcatcttcct gggcgatggg 180
atgggggtgt ctacggtgac agctgccagg atcctaaaag ggcagaagaa ggacaaactg 240
gggcctgaga tacccctggc catggaccgc ttcccatatg tggctctgtc caagacatac 300
aatgtagaca aacatgtgcc agacagtgga gccacagcca cggcctacct gtgcggggtc 360
aagggcaact tccagaccat tggcttgagt gcagccgccc gctttaacca gtgcaacacg 420
acacgcggca acgaggtcat ctccgtgatg aatcgggcca agaaagcagg gaagtcagtg 480
ggagtggtaa ccaccacacg agtgcagcac gcctcgccag ccggcaccta cgcccacacg 540
gtgaaccgca actggtactc ggacgccgac gtgcctgcct cggcccgcca ggaggggtgc 600
caggacatcg ctacgcagct catctccaac atggacattg acgtgatcct aggtggaggc 660
cgaaagtaca tgtttcgcat gggaacccca gaccctgagt acccagatga ctacagccaa 720
ggtgggacca ggctggacgg gaagaatctg gtgcaggaat ggctggcgaa gcgccagggt 780
gcccggtatg tgtggaaccg cactgagctc atgcaggctt ccctggaccc gtctgtgacc 840
catctcatgg gtctctttga gcctggagac atgaaatacg agatccaccg agactccaca 900
ctggacccct ccctgatgga gatgacagag gctgccctgc gcctgctgag caggaacccc 960
cgcggcttct tcctcttcgt ggagggtggt cgcatcgacc atggtcatca tgaaagcagg 1020
gcttaccggg cactgactga gacgatcatg ttcgacgacg ccattgagag ggcgggccag 1080
ctcaccagcg aggaggacac gctgagcctc gtcactgccg accactccca cgtcttctcc 1140
ttcggaggct accccctgcg agggagctcc atcttcgggc tggcccctgg caaggcccgg 1200
gacaggaagg cctacacggt cctcctatac ggaaacggtc caggctatgt gctcaaggac 1260
ggcgcccggc cggatgttac cgagagcgag agcgggagcc ccgagtatcg gcagcagtca 1320
gcagtgcccc tggacgaaga gacccacgca ggcgaggacg tggcggtgtt cgcgcgcggc 1380
ccgcaggcgc acctggttca cggcgtgcag gagcagacct tcatagcgca cgtcatggcc 1440
ttcgccgcct gcctggagcc ctacaccgcc tgcgacctgg cgccccccgc cggcaccacc 1500
gacgccgcgc acccgggtta ctctagagtc ggggcggccg gccgcttcga gcagacat 1558

<210> 27
<211> 1651
<212> DNA
<213> Künstliche Sequenz

<220>
<223> Beschreibung der künstlichen Sequenz: Luciferase
      Firefly

<400> 27
catggaagac gccaaaaaca taaagaaagg cccggcgcca ttctatccgc tggaagatgg 60
aaccgctgga gagcaactgc ataaggctat gaagagatac gccctggttc ctggaacaat 120
tgcttttaca gatgcacata tcgaggtgga catcacttac gctgagtact tcgaaatgtc 180
cgttcggttg gcagaagcta tgaaacgata tgggctgaat acaaatcaca gaatcgtcgt 240
atgcagtgaa aactctcttc aattctttat gccggtgttg ggcgcgttat ttatcggagt 300
tgcagttgcg cccgcgaacg acatttataa tgaacgtgaa ttgctcaaca gtatgggcat 360
ttcgcagcct accgtggtgt tcgtttccaa aaaggggttg caaaaaattt tgaacgtgca 420
aaaaaagctc ccaatcatcc aaaaaattat tatcatggat tctaaaacgg attaccaggg 480
atttcagtcg atgtacacgt tcgtcacatc tcatctacct cccggtttta atgaatacga 540
ttttgtgcca gagtccttcg atagggacaa gacaattgca ctgatcatga actcctctgg 600
atctactggt ctgcctaaag gtgtcgctct gcctcataga actgcctgcg tgagattctc 660
gcatgccaga gatcctattt ttggcaatca aatcattccg gatactgcga ttttaagtgt 720
tgttccattc catcacggtt ttggaatgtt tactacactc ggatatttga tatgtggatt 780
tcgagtcgtc ttaatgtata gatttgaaga agagctgttt ctgaggagcc ttcaggatta 840
caagattcaa agtgcgctgc tggtgccaac cctattctcc ttcttcgcca aaagcactct 900
gattgacaaa tacgatttat ctaatttaca cgaaattgct tctggtggcg ctcccctctc 960
taaggaagtc ggggaagcgg ttgccaagag gttccatctg ccaggtatca ggcaaggata 1020
tgggctcact gagactacat cagctattct gattacaccc gagggggatg ataaaccggg 1080
cgcggtcggt aaagttgttc cattttttga agcgaaggtt gtggatctgg ataccgggaa 1140
aacgctgggc gttaatcaaa gaggcgaact gtgtgtgaga ggtcctatga ttatgtccgg 1200
ttatgtaaac aatccggaag cgaccaacgc cttgattgac aaggatggat ggctacattc 1260
tggagacata gcttactggg acgaagacga acacttcttc atcgttgacc gcctgaagtc 1320
tctgattaag tacaaaggct atcaggtggc tcccgctgaa ttggaatcca tcttgctcca 1380
acaccccaac atcttcgacg caggtgtcgc aggtcttccc gacgatgacg ccggtgaact 1440
tcccgccgcc gttgttgttt tggagcacgg aaagacgatg acggaaaaag agatcgtgga 1500
ttacgtcgcc agtcaagtaa caaccgcgaa aaagttgcgc ggaggagttg tgtttgtgga 1560
cgaagtaccg aaaggtctta ccggaaaact cgacgcaaga aaaatcagag agatcctcat 1620
aaaggccaag aagggcggaa agatcgccgt g                                1651


<210> 28
<211> 658
<212> DNA
<213> Künstliche Sequenz

<220>
<223> Beschreibung der künstlichen Sequenz: CAT

<400> 28
atggagaaaa aaatcactgg atataccacc gttgatatat cccaatggca tcgtaaagaa 60
cattttgagg catttcagtc agttgctcaa tgtacctata accagaccgt tcagctggat 120
attacggcct ttttaaagac cgtaaagaaa aataagcaca agttttatcc ggcctttatt 180
cacattcttg cccgcctgat gaatgctcat ccggaactcc gtatggcaat gaaagacggt 240
gagctggtga tatgggatag tgttcaccct tgttacaccg ttttccatga gcaaactgaa 300
acgttttcat cgctctggag tgaataccac gacgatttcc ggcagtttct acacatatat 360
tcgcaagatg tggcgtgtta cggtgaaaac ctggcctatt tccctaaagg gtttattgag 420
aatatgtttt tcgtctcagc caatccctgg gtgagtttca ccagttttga tttaaacgtg 480
gccaatatgg acaacttctt cgcccccgtt ttcacgatgg gcaaatatta tacgcaaggc 540
gacaaggtgc tgatgccgct ggcgattcag gttcatcatg ccgtttgtga tggcttccat 600
gtcggcagaa tgcttaatga attacaacag tactgcgatg agtggcaggg cggggcgt   658


<210> 29

<211> 582
<212> DNA
<213> Künstliche Sequenz

<220>
<223> Beschreibung der künstlichen Sequenz: hGH

<400> 29
atgttcccaa ctattccact gagtcgcctg ttcgataacg cgatgctgcg tgcgcatcgt 60
ctgcaccaac tggctttcga cacttaccag gagttcgaag aagcatacat cccgaaagaa 120
cagaaataca gcttccttca gaacccacag acctcgttgt gtttctctga aagtatcccg 180
accccttcta accgcgaaga gacccagcag aaatcgaacc ttgaactgct tcgtatctcg 240
ctgcttctca ttcagtcgtg gctggagcca gtacagttcc tgcgttcggt tttcgcaaac 300
tcactggtat acggtgcgtc tgacagtaac gtttacgacc tgctgaaaga ccttgaagaa 360
gggatccaga ccctgatggg tcgcctggaa gatggttcac cacgcactgg tcagatcttc 420
aaacagactt actccaaatt cgatactaac tctcataacg atgatgctct gctgaaaaac 480
tacggcctgc tgtactgttt ccgtaaagat atggataaag ttgaaacttt cctgcgtatc 540
gttcagtgtc gttctgttga agggtcgtgt ggcttctaat ag 582


<210> 30
<211> 119
<212> DNA
<213> Oryctolagus cuniculus

<400> 30
tataaataaa cccctctgct aaccatgttc atgccttctt ctttttccta cagctcctgg 60
gcaacgtgct ggttgttgtg ctgtctcatc attttggcaa agaattcact cctcaggtg 119


<210> 31
<211> 802
<212> DNA
<213> Gallus gallus

<400> 31
ccctttgtgc gggggggagc ggctcggggg gtgcgtgcgt gtgtgtgtgc gtggggagcg 60
ccgcgtgcgg cccgcgctgc ccggcggctg tgagcgctgc gggcgcggcg cggggctttg 120
tgcgctccgc gtgtgcgcga ggggagcgcg gccgggggcg gtgccccgcg gtgcgggggg 180
gctgcgaggg gaacaaaggc tgcgtgcggg gtgtgtgcgt gggggggtga gcagggggtg 240
tgggcgcggc ggtcgggctg taaccccccc ctgcaccccc ctccccgagt tgctgagcac 300
ggcccggctt cgggtgcggg gctccgtgcg gggcgtggcg cggggctcgc cgtgccgggc 360
ggggggtggc ggcaggtggg ggtgccgggc ggggcggggc cgcctcgggc cggggagggc 420
tcgggggagg ggcgcggcgg ccccggagcg ccggcggctg tcgaggcgcg gcgagccgca 480
gccattgcct tttatggtaa tcgtgcgaga gggcgcaggg acttcctttg tcccaaatct 540
ggcggagccg aaatctggga ggcgccgccg caccccctct agcgggcgcg ggcgaagcgg 600
tgcggcgccg gcaggaagga aatgggcggg gagggccttc gtgcgtcgcc gcgccgccgt 660
ccccttctcc atctccagcc tcggggctgc cgcaggggga cggctgcctt cgggggggac 720
ggggcagggc ggggttcggc ttctggcgtg tgaccggcgg ggtttatatc ttcccttctc 780
tgttcctccg cagccagcca tg 802


<210> 32
<211> 1032
<212> DNA
<213> Bacteriophage P1

<400> 32
atgtccaatt tactgaccgt acaccaaaat ttgcctgcat taccggtcga tgcaacgagt 60

gatgaggttc gcaagaacct gatggacatg ttcagggatc gccaggcgtt ttctgagcat 120
acctggaaaa tgcttctgtc cgtttgccgg tcgtgggcgg catggtgcaa gttgaataac 180
cggaaatggt ttcccgcaga acctgaagat gttcgcgatt atcttctata tcttcaggcg 240
cgcggtctgg cagtaaaaac tatccagcaa catttgggcc agctaaacat gcttcatcgt 300
cggtccgggc tgccacgacc aagtgacagc aatgctgttt cactggttat gcggcggatc 360
cgaaaagaaa acgttgatgc cggtgaacgt gcaaaacagg ctctagcgtt cgaacgcact 420
gatttcgacc aggttcgttc actcatggaa aatagcgatc gctgccagga tatacgtaat 480
ctggcatttc tggggattgc ttataacacc ctgttacgta tagccgaaat tgccaggatc 540
agggttaaag atatctcacg tactgacggt gggagaatgt taatccatat tggcagaacg 600
aaaacgctgg ttagcaccgc aggtgtagag aaggcactta gcctgggggt aactaaactg 660
gtcgagcgat ggatttccgt ctctggtgta gctgatgatc cgaataacta cctgttttgc 720
cgggtcagaa aaaatggtgt tgccgcgcca tctgccacca gccagctatc aactcgcgcc 780
ctggaaggga tttttgaagc aactcatcga ttgatttacg gcgctaagga tgactctggt 840
cagagatacc tggcctggtc tggacacagt gcccgtgtcg gagccgcgcg agatatggcc 900
cgcgctggag tttcaatacc ggagatcatg caagctggtg gctggaccaa tgtaaatatt 960
gtcatgaact atatccgtaa cctggatagt gaaacagggg caatggtgcg cctgctggaa 1020
gatggcgatt ag 1032

<210> 33
<211> 34
<212> DNA
<213> Bacteriophage P1

<400> 33
ataacttcgt ataatgtatg ctatacgaag ttat 34

<210> 34
<211> 384
<212> DNA
<213> Streptomyces avidinii

<400> 34
gctgctgaag caggtatcac cggcacctgg tacaaccagc tcggctcgac cttcatcgtg 60
accgcgggcg ccgacggcgc cctgaccgga acctacgagt cggccgtcgg caacgccgag 120
agccgctacg tcctgaccgg tcgttacgac agcgccccgg ccaccgacgg cagcggcacc 180
gccctcggtt ggacggtggc ctggaagaat aactaccgca acgcccactc cgcgaccacg 240
tggagcggcc agtacgtcgg cggcgccgag gcgaggatca acacccagtg gctgctgacc 300
tccggcacaa ccgaggccaa cgcctggaag tccacgctgg tcggccacga caccttcacc 360
aaggtgaagc cgtccgccgc ctcc 384

<210> 35
<211> 42
<212> DNA
<213> Künstliche Sequenz

<220>
<223> Beschreibung der künstlichen Sequenz: myc epitop

<400> 35
ggatccgaac aaaagctgat ctcagaagaa gatctatgca ta 42

<210> 36
<211> 18
<212> DNA
<213> Künstliche Sequenz

<220>
<223> Beschreibung der künstlichen Sequenz: 5xHis
      Sequenz

<400> 36
catcaccatc atcattaa                                                18


<210> 37
<211> 29
<212> DNA
<213> Künstliche Sequenz

<220>
<223> Beschreibung der künstlichen Sequenz: Primer

<400> 37
gtccttaagc ttgatatcaa gggtcagcg                                    29


<210> 38
<211> 29
<212> DNA
<213> Künstliche Sequenz

<220>
<223> Beschreibung der künstlichen Sequenz: Primer

<400> 38
ggcttctgaa ttctgggtgg gtggtgtct                                    29


<210> 39
<211> 19
<212> DNA
<213> Künstliche Sequenz

<220>
<223> Beschreibung der künstlichen Sequenz: Primer

<400> 39
tgttctcagg ctcaggatg                                               19


<210> 40
<211> 25
<212> DNA
<213> Künstliche Sequenz

<220>
<223> Beschreibung der künstlichen Sequenz: Primer

<400> 40
ctgtttcgag agagcaaagc ttagg                                        25


<210> 41

<211> 18
<212> DNA
<213> Künstliche Sequenz

<220>
<223> Beschreibung der künstlichen Sequenz: Primer

<400> 41
gagtgaacgg ccagcttc                                                    18

## Beschreibung

### Gebiet der Erfindung

Die Erfindung betrifft spezifische, regulatorische Sequenzbereiche des alpha Smooth Muscle Aktin-Gens (α-SMA-Gens), Fusionskonstrukte, in denen solche regulatorische Sequenzbereiche des α-SMA-Gens mit weiteren funktionellen Nukleinsäuresequenzen operativ verknüpft sind, sowie die Verwendung solcher regulatorischer Sequenzbereiche zur zelltyp- und differenzierungspezifischen Reporterexpression, zu zelltyp- und differenzierungsspezifischen Genveränderungen (Alteration, Mutation) und zu Genexpressionsveränderungen in Zellen und Organismen.

Im einzelnen betrifft die vorliegende Erfindung Fusionskonstrukte, in denen der genannte spezifische Sequenzbereich, der im wesentlichen aus Sequenzen besteht, die sich im 5'-terminalen Bereich des α-SMA-Gens befinden, mit einer weiteren funktionellen Nukleinsäuresequenz, vorzugsweise mit peptid- oder proteinkodierenden Nukleinsäuresequenzen, regulatorischen DNA-Sequenzen oder funktionellen RNA-kodierenden Sequenzen operativ verknüpft ist; ein Verfahren, in dem die oben genannten Fusionskonstrukte in einen Vektor operativ inseriert werden und das Vektorkonstrukt in eukaryontische Zellen eingebracht wird; ein Verfahren, in dem die mit dem Vektorkonstrukt transient oder stabil transfizierten, transformierten oder infizierten Zellen, einen Reporter unter Kontrolle der genannten regulatorischen Sequenzen des α-SMA-Gens exprimieren und die Reporterexpression zur Isolierung oder zur Sichtung von Glatten Muskelzellen, Myofibroblasten oder myofibroblastähnlichen Zellen aus einem Zellgemisch, einer Zellpopulation, einem Zellaggregat oder einem Organismus genutzt wird; sowie ein Verfahren, bei dem mittels der genannten Fusionskonstrukte (die regulatorische Sequenzen des α-SMA-Gens und peptid- oder proteinkodierende bzw. RNA-kodierende Sequenzen enthalten) die Genexpression bzw. die Zellfunktion von Glatten Muskelzellen oder Myofibroblasten in Organismen bzw. in Organen unter Nutzung transgener oder knock-out /-in Technologie oder gentherapeutischer Fusionsvektorapplikation manipuliert werden.

### Hintergrund der Erfindung

Das α-SMA-Gen ist ein Mitglied der Aktin-Multigenfamilie, die für verschiedene Isoformen der zytoskeletalen Aktine kodiert. Die Expression der verschiedenen Aktine ist in hohem Grade zelltypspezifisch und differenzierungsabhängig reguliert. So wird adult das skelettale α-Aktin in der Skelettmuskulatur, das cardiale α-Aktin in der Herzmuskulatur und das α-SMA in der glatten Muskulatur exprimiert. Die transkriptionelle Regulation des α-SMA-Gens wird durch ein Zusammenspiel von positiv und negativ wirkenden regulatorischen Elementen des 5'-Bereiches vermittelt (Blank, R. S. et al., J. Biol. Chem. 267: 984-989 (1992); Carrol, S. L. et al., J. Biol. Chem. 261: 8955-8976 (1986); Carrol, S. L. et al., Mol. Cell. Biol. 8: 241-250 (1988); Nakano, Y. et al., Gene. 99: 275-289 (1991)). Der 5'-Bereich des α-SMA-Gens enthält verschiedene konservierte *cis*-Elemente wie zwei CArG-Elemente (CArG-A und B) (Carrol, S. L. et al., Mol. Cell. Biol. 8: 241-250 (1988) und Blank, R. S. et al., J. Biol. Chem. 267: 984-989 (1992)). Die CArG-Motive bestehen aus einem A/T Repeat, das von CC/GG flankiert wird, und werden als maßgebliche Elemente der muskelspezifischen Genregulation diskutiert (Chow, K. L., Schwartz, R. J., Mol. Cell. Biol., 10, 528 - 538 (1990) und Sobuc, K. et al., Mol. Cell. Biochem. 190:105-118 (1999)). Reporterstudien an trangenen Reportermäusen zeigten, daß eine weitere CArG-Box (0) im ersten Intron des α-SMA-Gens für eine *in vivo* Expression von α-SMA in glatten Muskelzellen notwendig ist (Mack, C. P., Owens, G. K., Circ. Res., 84: 852 - 861 (1999)).

Der 5'-Bereich des α-SMA-Gens enthält ebenfalls zwei E-Box Konsensussequenzen (CA NN TG), potentielle Bindestellen für basic helix-loop-helix Transkriptionsfaktoren (Johnson, A. D., Owens, G. K., Am. J. Physiol., 276, C1420 - C1431 (1999)) und weitere Konsensussequenzen (Hautmann, M. B. et al., J. Biol. Chem., 272: 10948-10956 (1997) und McNamara, C. A. et al., Am. J. Physiol., 268: C1259-C1266 (1995)), deren Bedeutung im einzelnen nicht geklärt ist.

Neben der zelltypspezifischen Expression in glatten Muskelzellen (Vanderkerckhove J. and Weber K., Differentiation 14: 123-133 (1979) Skalli, O. et al., J. Cell. Biol., 103: 2787-2796 (1986) und idem, J. Histochem. Cytochem., 37: 315-321 (1989)) wird α-SMA transient in der Embryogenese während der Differenzierung von skelettalen und cardialen Muskelzellen (Ruzicka, D. L. et al., J. Cell Biol., 107: 25775 - 25786 (1988); Woodcock-Mitchell, J., Mitchell, J. J., Differentiation 39: 161 - 166 (1988)), aber auch in Myofibroblasten während der Wundheilung sowie nach myofibroblastischen Veränderungen verschiedener Zelltypen in Vernarbungs- und Gewebeumstrukturierungsprozessen nach chronischer Organschädigung exprimiert (Desmoulière A. et al., Exp. Nephrol. 3: 134-139 (1995); Gabbiani G., Cardiovasc. Res. 38: 545-548 (1998); idem, Pathol. Res. Pract. 190: 851-853 (1994); idem, Am. J. Pathol. 83: 457-474 (1976)).

Myofibroblasten sind Zelltypen mesodermalen Ursprungs, die sich durch eine ausgeprägte Matrixproduktion, ein heterogenes Intermediatfilamentmuster mit Desmin oder Vimentin auszeichnen und durch die α-SMA-Expression die Fähigkeit zur Kontraktilität besitzen (Desmoulière, A. et al., Exp. Nephrol. 3: 134-139 (1995); Gabbiani, G., Cardiovasc. Res. 38: 545-548 (1998); idem, Pathol. Res. Pract. 190: 851-853 (1994); idem, Am. J. Pathol. 83: 457-474 (1976)). Sie treten temporär bei der Wundheilung auf, während persistierende Myofibroblasten bei chronischen Vernarbungsprozessen an der Bindegewebs- und der Organumstrukturierung beteiligt sind. In verschiedenen Fibroseerkrankungen aufgrund chronischer Organschädigung wie der Hepatitis, der Glomerulonephritis oder fibrosierenden Lungenerkrankungen sind Myofbroblasten die Hauptmatrixproduzenten (Friedman, S. L., New. Engl. J. Med. 328: 1828-35 (1993); Gressner, A. M., Kidney Int. 49: 39-45 (1996); MacPherson, B. R. et al., Hum. Pathol., 24:710 - 716 (1993); Alpers, C. E. et al., Kidney Int. 41: 1134-1142 (1992); Johnson, R. J. et al., J. Clin. Invest. 87: 847-858 (1991); Kapanci, Y. et al., Mod. Pathol. 10: 1134-42 (1997)). Daher wurden Myofibroblasten auch als modulierte Fibroblasten bezeichnet; ihre Vorläuferzelltypen sind jedoch abhängig vom Organtyp sehr unterschiedlich. Chronische Nierenschädigungen führen zu einer Transdifferenzierung von Mesangialzellen zu myofibroblastischen Zellen (Johnson R. J. et al., J. Clin. Invest. 87: 847-858 (1991); MacPherson B. R. et al., Hum. Pathol. 24: 710-716 (1993)). In der Leber kommt es nach chronischer Schädigung unabhängig, ob die Ursache chronischer Alkoholabusus, virale Hepatitis B oder C-Infektionen oder andere chronische Schädigungen sind, zur Leberfibrose, in der Hepatische Sternzellen (HSC) zu Myofibroblasten transdifferenzieren (Friedman S. L., New. Engl. J. Med. 328: 1828-35 (1993); Gressner A.M., Kidney Int. 49: 39-45 (1996); Ramadori G. et al., Virch. Arch. [B] 59: 349-357 (1990)). Hepatische Sternzellen besitzen perizytäre Eigenschaften und speichern im ruhenden Zustand 80 % des körpereigenen Vitamin A (Friedman S. L., New. Engl. J. Med. 328: 1828-35 (1993)). Nach myofibroblastischer Transdifferenzierung, die neben der Matrixproduktion und der α-SMA-Expression vermittelten Kontraktilität durch eine einsetzende Proliferation, Vitamin A-Verlust und ein verändertes Wachstumsfaktorenprofil gekennzeichnet ist, sind sie maßgeblich an der Aufrechterhaltung und dem Fortlauf der Leberfibrose beteiligt (Friedman, S. L., New. Engl. J. Med. 328: 1828-35 (1993); Gressner, A. M., Kidney Int. 49: 39-45 (1996)).
Beim Übergang von *in situ* Karzinomen in invasive Karzinome treten ebenfalls Myofibroblasten mit den typischen Eigenschaften von induzierter extrazellulärer Matrixproduktion und Kontraktilität auf (Cintorino, M. et al., Int. J. Cancer 47: 832 - 836 (1984); Gabbiani, G. et al., Am. J. Pathol. 83:457 - 474 (1976)).

Gentherapeutische Ansätze für chronische Fibroseerkrankungen: Da Myofibroblasten die zentralen Zelltypen in vielen sklerosierenden Erkrankungen sind, sind sie ideale Zielzellen der Therapie, auch der Gentherapie. Durch eine zelltypspezifische, differenzierungsabhängige Kontrolle der Expression
(a) von Genen kodierend für z.B. intrazellulären Signalmediatoren (Walther, W., Stein, U., Mol. Biotechnol. 13(1): 21 - 28 (1999)) oder
(b) RNA-kodierenden Sequenzbereiche, die durch Anlagerung die Transkription oder die Translation bestimmter Gene beinflussen (Bai, J. et al., Mol. Ther. 1(3): 244 - 254 (2000)) oder
(c) eines Enzyms für eine zyklische Rekombination (CRE), das floxP-flankierte Genbereiche verändert (Sauer, B. Methods 14: 381 - 392 (2998)),
wird eine Gentherapie bestimmter Zielzellen ermöglicht. Als Vekoren werden vielfach virale Vesikel wie z.B. adenovirale oder retrovirale Abkömmlinge (Miller, N. Whelan, J. Hum. Gene Ther. 8, 803 - 815; Mountain, A., Tibtech. 18: 119 - 128 (2000); Schiedner, G. et al., Nat. Genet. 18, 180 - 183 (1998); Parr, M. J., Nat. Med. 3, 1145 - 1149 (1997); Ory, D. S. et al., Proc. Natl. Acad. Sci. 93:11400 - 11406 (1996); Feng, M. et al., Nat. Biotechnol. 15: 866 - 870 (1997)) benutzt. Aber auch die DNA eines Plasmidkonstruktes kann blank oder in Aggregaten mit Liposomen oder Polymerkörpern verabreicht werden (Hengge, U. et al., Nat. Genet. 10:161 - 166 (1995); Gottschalk, S. et al., Gene Ther. 3:3410 - 3414 (1990); Wagner, E. et al., Proc. Natl. Acad. Sci. 87:3410 - 3414 (1990)). Für gentherapeutische Ansätze und Veränderungen der myofibroblastischen Zellfunktion sind geeignete regulatorische Sequenzen bzw. ein Promoter notwendig, die/der die entsprechenden Zellfunktionseingriffe und Veränderungen in der Expression in Myofibroblasten dirigieren kann, voraussetzende Bedingung, die aber bislang nicht erfüllt ist. Denn erst durch den Einsatz regulatorischer Sequenzen innerhalb eines Promoters, die die Zellfunktionseigenschaften (a) durch eine veränderte Genexpression, (b) durch eine Einwirkung in die Proteinsynthese oder (c) durch Genomalteration myofibroblastensprechend dirigieren, können myofibroblastische Zellen gentherapeutisch angesprochen werden.

### Zusammenfassung der Erfindung

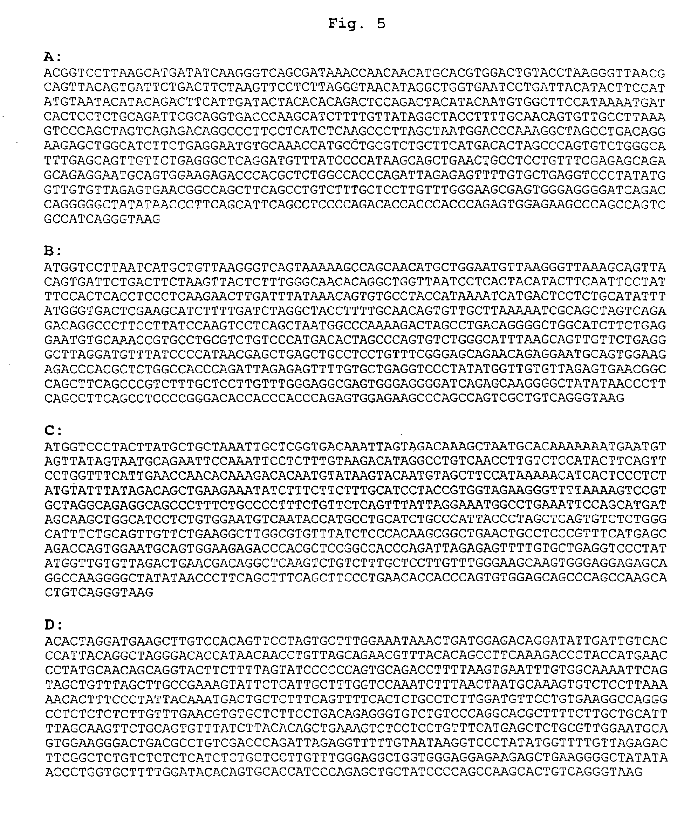

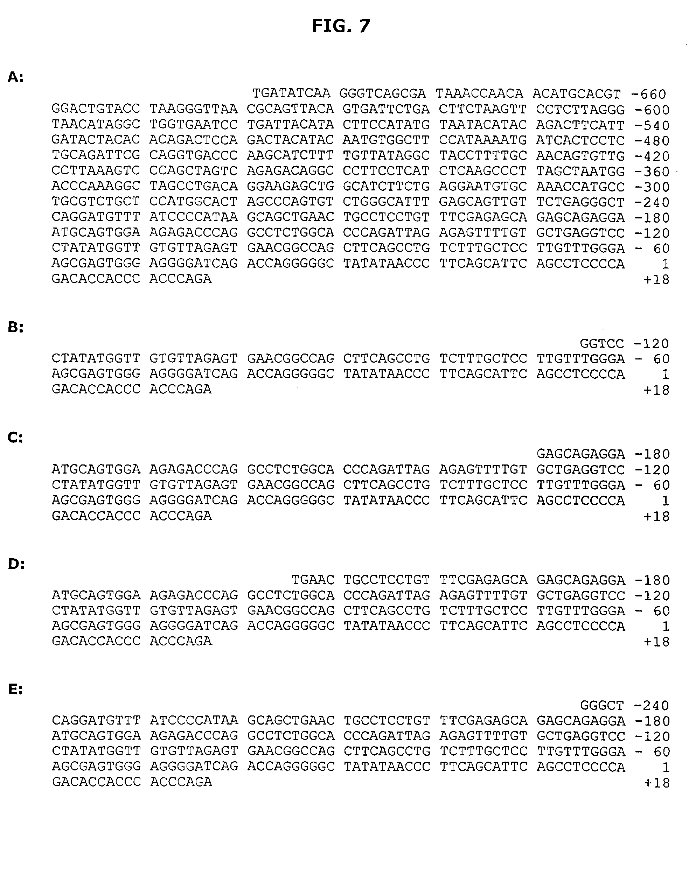

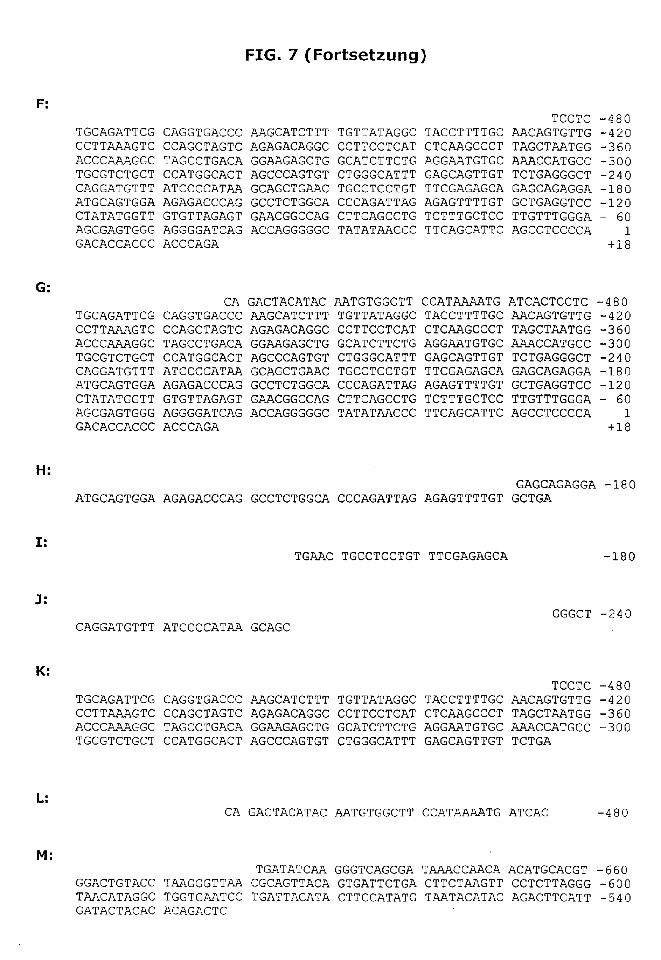

Bei der Charakterisierung des regulierenden Sequenzbereichs des α-SMA-Gens (nachfolgend auch "α-SMA-5'-Sequenzbereich") wurde überraschenderweise gefunden, dass einzelne Bereiche hierin, nämlich innerhalb -698 bis +18 des α-SMA-5'-Sequenzbereichs (Nummerierung, falls nicht anders angegeben, bezogen auf das in Fig. 7A gezeigte α-SMA-Gen aus rattus norvegicus) Aktivitäten aufweisen, die sich von der Aktivität des Gesamtbereiches unterscheiden. So wurde gefunden, dass der Bereich -189 bis +18 des α-SMA-Gens transkriptionsaktivierend ist, wohingegen der Bereich -698 bis -190 zelltypspezifische und differenzierungsabhängige regulative Eigenschaften aufweist, d. h. in Verbindung mit dem Core-Bereich (-189 bis +18) für die zelltypspezifische Kontrolle zuständig ist. Aufbauend auf den Befund, dass diese Bereiche die Genexpression in Myofibroblasten und myofibroblastähnlichen Zellen oder auch in anderen transient oder permanent SMA-exprimierenden Zellen steuern können, wurde ein System für eine myofibroblastischen Steuerung erarbeitet. Die vorliegende Erfindung betrifft demgemäss
(1) eine Nukleinsäuresequenz, umfassend
   (i) einen oder mehrere funktionelle Bereiche aus dem regulierenden Sequenzbereich des alpha-Smooth-Muscle-Actin-Gens (α-SMA-Gens) und
   (ii) wenigstens eine weitere funktionelle Nukleinsäuresequenz, die mit der Sequenz (i) operativ verknüpft ist;
(2) eine bevorzugte Ausführungsform der in (1) definierten Nukleinsäuresequenz, wobei der regulierende Sequenzbereich von der Ratte stammt und die Nukleinsäuresequenz vorzugsweise einen oder mehrere funktionelle Bereiche aus der in Fig. 5A gezeigten Sequenz (SEQ ID NO:1), insbesondere aus der in Fig. 7A gezeigten Sequenz (SEQ ID NO:5), umfasst;
(3) eine bevorzugte Ausführungsform der in (2) definierten Nukleinsäuresequenz, wobei der funktionelle Bereich eine transkriptionsaktivierende Nukleinsäuresequenz ist und insbesondere aus der Sequenz -189 bis +18 der Fig. 7A (d.h. aus der Sequenz der Fig. 7C; SEQ ID NO:7) stammt;
(4) eine bevorzugte Ausführungsform der in (2) definierten Nukleinsäuresequenz, wobei der funktionelle Bereich eine zelltypspezifische Nukleinsäuresequenz ist und insbesondere aus der Sequenz -698 bis -190, besonders bevorzugt aus der Sequenz -698 bis -215, der Fig. 7A stammt;
(5) eine bevorzugte Ausführungsform der in (1) definierten Nukleinsäuresequenz, wobei der regulierende Sequenzbereich von der Maus stammt und die Nukleinsäuresequenz vorzugsweise einen oder mehrere funktionelle Bereiche der in Fig. 5B gezeigten Sequenz (SEQ ID NO:2) insbesondere aus der in SEQ ID NO:18 gezeigten Sequenz aufweist;
(6) eine bevorzugte Ausführungsform der in (5) definierten Nukleinsäuresequenz, wobei der funktionelle Bereich
   (a) eine transkriptionsaktivierende Nukleinsäuresequenz ist und insbesondere aus den Nukleotiden 490 bis 697 der in SEQ ID NO:18 gezeigten Sequenz stammt und/oder
   (b) eine zelltypspezifische Nukleinsäuresequenz ist und insbesondere aus den Nukleotiden 1 bis 489 der in SEQ ID NO:18 gezeigten Sequenz stammt;
(7) eine bevorzugte Ausführungsform der in (1) definierten Nukleinsäuresequenz, wobei der regulierende Sequenzbereich vom Menschen stammt und die Nukleinsäuresequenz vorzugsweise einen oder mehrere funktionelle Bereiche der in Fig. 5C gezeigten Sequenz (SEQ ID NO:3) insbesondere aus der in SEQ ID NO:19 gezeigten Sequenz aufweist;
(8) eine bevorzugte Ausführungsform der in (7) definierten Nukleinsäuresequenz, wobei der funktionelle Bereich
   (a) eine transkriptionsaktivierende Nukleinsäuresequenz ist und insbesondere aus den Nukleotiden 513 bis 715 der in SEQ ID NO:19 gezeigten Sequenz stammt und/oder
   (b) eine zelltypspezifische Nukleinsäuresequenz ist und insbesondere aus den Nukleotiden 1 bis 512 der in SEQ ID NO:19 gezeigten Sequenz stammt;
(9) eine bevorzugte Ausführungsform der in (1) definierten Nukleinsäuresequenz, wobei der regulierende Sequenzbereich vom Huhn stammt und die Nukleinsäuresequenz vorzugsweise einen oder mehrere funktionelle Bereiche der in Fig. 5D gezeigten Sequenz insbesondere aus der in SEQ ID NO:20 gezeigten Sequenz aufweist;
(10) eine bevorzugte Ausführungsform der in (9) definierten Nukleinsäuresequenz, wobei der funktionelle Bereich
   (a) eine transkriptionsaktivierende Nukleinsäuresequenz ist und insbesondere aus den Nukleotiden 497 bis 699 der in SEQ ID NO:20 gezeigten Sequenz stammt und/oder
   (b) eine zelltypspezifische Nukleinsäuresequenz ist und insbesondere aus den Nukleotiden 1 bis 496 der in SEQ ID NO:20 gezeigten Sequenz stammt;
(11) eine transkriptionsaktivierende Nukleinsäuresequenz, wie in (3), (6), (8) oder (10) definiert;
(12) eine zelltypspezifische Nukleinsäuresequenz, wie in (4), (6), (8) oder (10) definiert;
(13) einen Vektor, enthaltend eine Nukleinsäuresequenz, wie in (11) und/oder (12) definiert;
(14) einen Vektor, in dem eine Nukleinsäuresequenz, wie in (1) bis (10) definiert, inseriert ist;
(15) eukaryontische Zellen oder Organismen, die mit einer Nukleinsäuresequenz, wie in (1) bis (10) definiert oder einem Vektor, wie in (13) oder (14) definiert, transient oder stabil transfiziert transformiert oder infiziert sind;
(16) ein Verfahren zur Herstellung der eukaryontischen Zellen, wie in (15) definiert, umfassend das Transfizieren, Transformieren oder Infizieren von Ausgangszellen mit den Nukleinsäuresquenzen, wie in (1) bis (10) definiert oder mit Vektoren, wie in (13) oder (14) definiert;
(17) ein Verfahren zur Herstellung der Organismen, wie in (15) definiert, umfassend das Injizieren von ES-Zellen, die mit den Nukleinsäuresquenzen, wie in (1) bis (10) definiert oder mit Vektoren, wie in (13) oder (14) definiert, transfiziert sind, in Blastozysten der Organismen und Einsetzen in die Leihmutter;
(18) ein Verfahren zur Isolierung oder zur Sichtung von Glatten Muskelzellen, Myofibroblasten oder myofibroblastähnlichen Zellen aus einem Zellgemisch, einer Zellpopulation, einem Zellaggregat oder einem Organismus, umfassend die Expression eines Reportergens in einer eukaryontischen Zelle oder einen Organismus, wie in (15) definiert;
(19) ein Verfahren zur Manipulation der Genexpression und/oder der Zellfunktion von glatten Muskelzellen oder Myofibroblasten in Organismen oder Organen, umfassend Einbringen von Nukleinsäuresequenzen, wie in (1) bis (10) definiert oder einem Vektor, wie in (14) definiert, in die Organismen oder Organe;
(20) ein Arzneimittel, enthaltend eine Nukleinsäuresequenz, wie in (1) bis (10), (11) oder (12) definiert, oder einen Vektor, wie in (13) oder (14) definiert und
(21) die Verwendung eines funktionellen Bereichs aus dem regulierenden Sequenzbereich des α-SMA-Gens zur zelltyp- und differenzierungsspezifischen Reporterexpression und zur zelltyp- und differenzierungsspezifischen Genveränderung.

Die Erfindung wird nachfolgend anhand der beigefügten Figuren und Beispiele näher erläutert.

### Figurenbeschreibung

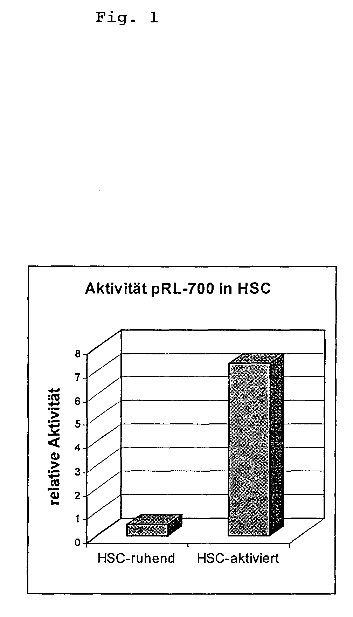

Fig 1: Messung der Luziferasereporteraktivität in ruhenden und myofibroblastischen (aktivierten) Hepatischen Sternzellen (HSC) unter Kontrolle des PRL-700-Reporterkonstruktes.

Fig 2a: Immuncytologie nach Einzelzelldissoziation von differenzierten EB und Kultivierung der vom Verband gelösten Zellen. In pSMA-GFP-190 transfizierten Zellen zeigten auch α-SMA-negative Zellen (1C) eine GFP-Expression (1B). In pSMA-GFP-700 transfizierten Zellen war die GFP-Expression (2B) auf α-SMA-exprimierenden Zellen begrenzt (2C).

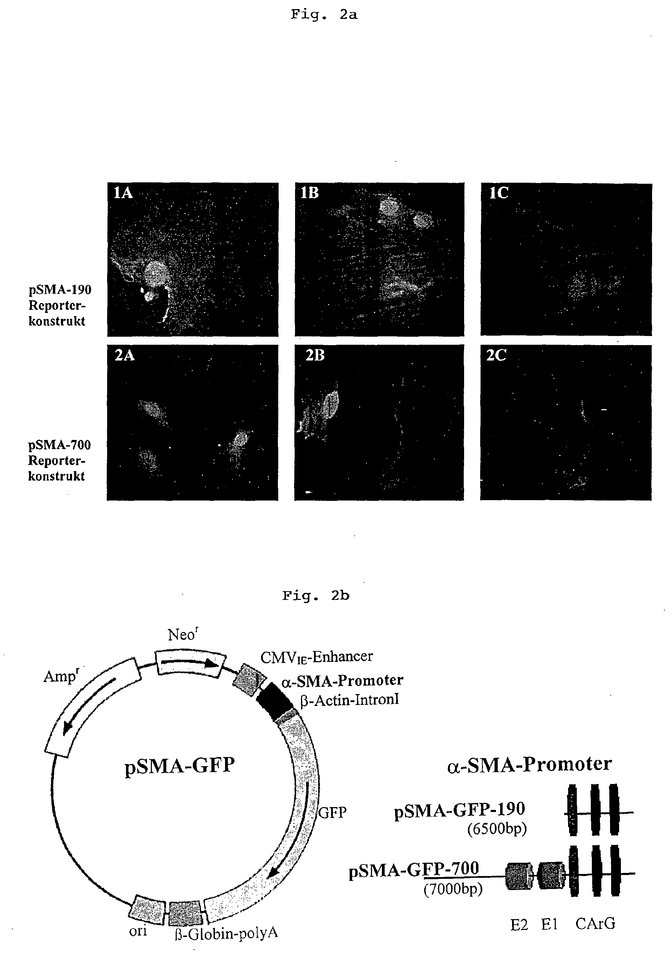

Fig 2b: Aufbau des SMA-GFP-700 bzw. SMA-GFP-190 Reportervektor für stabile Transfektionen in embryonale Stammzellen oder transgene Reportermausmodelle. In die Konstrukte ist zwischen den GFP-Reporter (EGFP-Variante) und den 716bp (SEQ ID NO:5) bzw. 207bp (SEQ ID NO:7) aus der 5'-Region des α-SMA-Gen als heterologe regulatorische Sequenz das erste Intron des chicken β-Actins kloniert. Zur Selektion von stabilen Transfektanten hat das Konstrukt, neben der Ampicillinresistenz eine weitere Resistenz gegen Neomycin, die in eukaryotischen Zellen eine G418 Selektion erlaubt.

Fig 3a: Luciferasereporteraktivitäten unterschiedlich langer SMA-Konstrukte (SMA-125 bis SMA-700; vergleiche auch Fig. 7) die nach transienter Transfektion in embryonalen Myotuben und Myoblasten der Zellinie L6 ermittelt wurden. L6-Zellen, die nach Serumentzug in α-SMA-exprimierende Myotuben differenzieren, zeigten eine 4- bis 5fache Induktion aller Konstrukte im Vergleich zu den Vorläufer L6-Myoblasten. Das SMA-190-Konstrukt besitzt einen stark transaktivierenden Einfluss. Das SMA-700-Konstrukt dirigiert dagegen eine differenzierungsabhängige Reporterexpression. Die Aktivitäten der SMA-Konstrukte wurden mit der Aktivität des SV40-Promoter (100%) in Relation gesetzt. Die Versuche wurden in drei unabhängigen Experimenten mit Doppelansätzen durchgeführt.

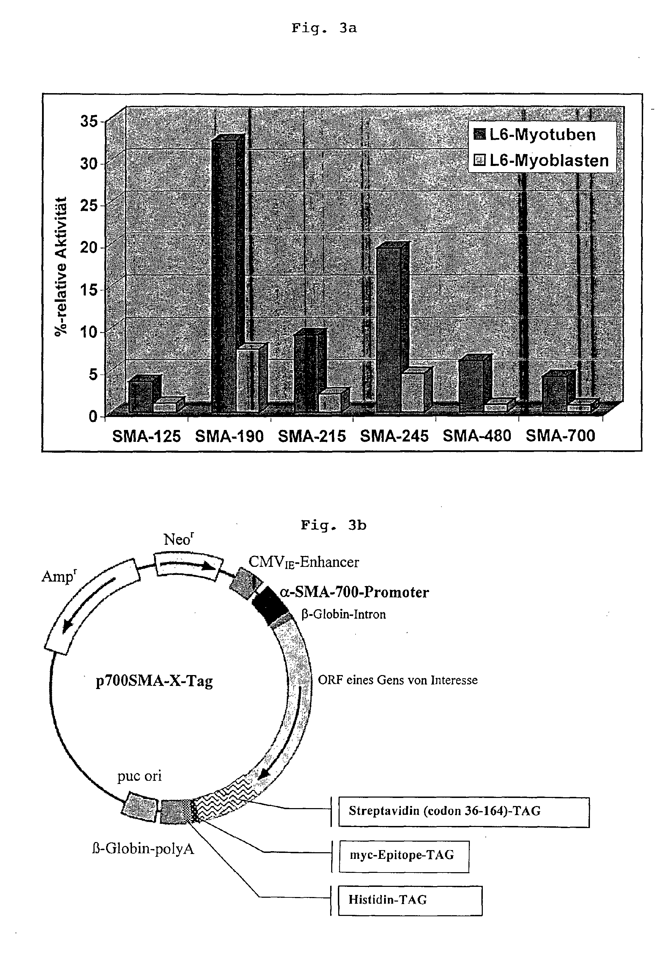

Fig 3b: Plasmidkonstrukt zur Expression eines Peptids oder Polypeptids von Interesse unter Kontrolle der 716 bp-5'-Sequenz (SEQ ID NO:5) des α -SMA-Gens. Zur Detektion der Expression kann in den Consensus des Leserasters (ORF) ein oder mehrere Erkennungssequenzen (Tags) eingebaut werden wie die kodierende Sequenz für Streptavidin (SEQ ID NO:34) und/oder ein von einem Antikörper (9E10) erkanntes Epitop wie myc (SEQ ID:35), und/oder zur Aufreinigung auch eine für fünf Histidine kodierende Sequenz (SEQ ID NO:36) enthalten. Sowohl die Sequenz des Peptids von Interesse als auch die damit verknüpften TAG-Sequenzen unterliegen der zelltypspezifischen und differenzierungsabhängigen Kontrolle des regulativen Sequenzbereichs des α-SMA-Gens.

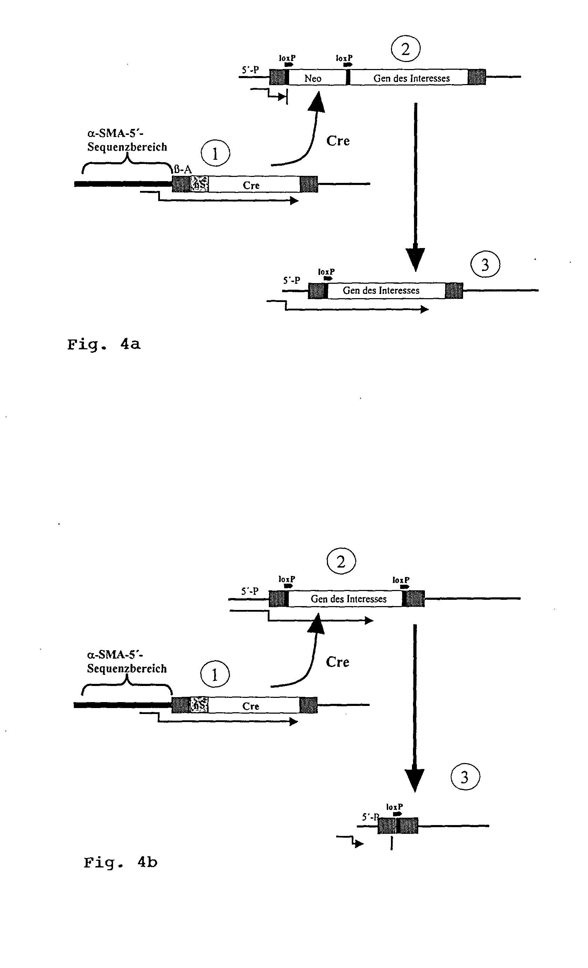

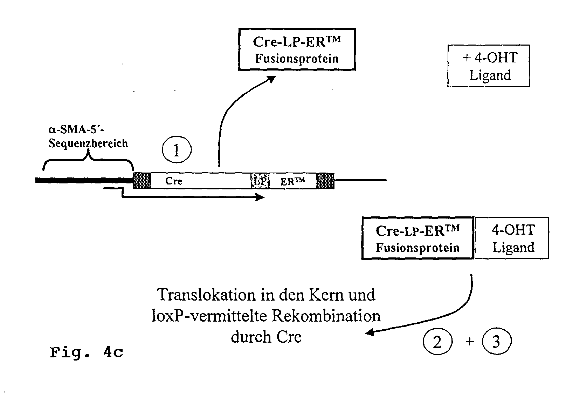

Fig 4: LoxP-vermittelte Cre-Rekombination unter Kontrolle des α-SMA-5'-Genbereichs:
a.: Aufgrund der transkriptionellen Kontrolle des Cre-Gens (SEQ ID NO:32) durch den 5'-Bereich des α-SMA-5'-Sequenzbereichs (SEQ ID NO:5) in Kombination mit dem ersten Intron des β-Actins (SEQ ID NO:31) kommt es nach myofibroblastischer Differenzierung zur Expression des Cre-Gens in Myofibroblasten (1), der Cre-vermittelten Excision des loxP-flankierten Neomycin-Gens (neo) (2) und der anschließenden Expression des Gens von Interesse (3).
b.: Aufgrund der transkriptionellen Kontrolle des Cre-Gens (SEQ ID NO:32) durch den 5'-Bereich des a-SMA-5'-Sequenzbereichs (SEQ ID NO:5) in Kombination mit dem ersten Intron des β-Actins (SEQ ID NO:31) kommt es nach myofibroblastischer Differenzierung zur Expression des Cre-Gens in Myofibroblasten (1), der Cre-vermittelten Excision des loxP-flankierten Gens von Interesse (2), sodaß eine anschließende Expression des Gens von Interesse unterbrochen wird (3).
c.: Aufgrund der transkriptionellen Kontrolle des Cre-ER-Fusionsgens (SEQ ID NO:32 +ER™, Danielian PS et al., Mol. Endocrinol. 7: 232-240, 1993) durch den 5'-Bereich des a-SMA-5'-Sequenzbereichs (SEQ ID NO:5) in Kombination mit dem ersten Intron des β-Actins (SEQ ID NO:31) kommt es nach myofibroblastischer Differenzierung zur Expression des Cre-ER-Fusionsgens in Myofibroblasten (1). Nach Zusatz bzw. Injektion von 0,1 bis 2 mg 4-OHT kann im nächsten Schritt die Bindung des Liganden durch das ER-Peptid erfolgen und sich ein Cre-ER/4-OHT-Komplex bilden. Anschließend transloziert der Kompex in den Kern und es werden durch Cre die in Fig. 4a und b gezeigten genetischen Veränderungsschritte (2) und (3) durchgeführt.

In Fig. 4 bedeutet:
nS: nukleäre Signalpeptidsequenz
schraffierte Fläche: Intronsequenz
LP: Linker Peptidsequenz
ER™:Sequenz für alterierten Östrogenrezeptor mit hoher Affinität für den synthetischen Liganden, 4-Hydroxytamoxifen (4-OHT), der aber endogen produziertes 17β-Estradiol nicht bindet.
β-A: β-Aktin-Intron (SEQ ID NO:31)
4-OHT: 4-Hydroxytamoxifen

Fig 5: 5'-upstream Region und Anfang Exon1 des α-SMA in verschiedenen Spezien (Region -713 bis +52, Nummerierung bezogen auf Sequenz A)
A: Ratte (Rattus norvegicus; SEQ ID NO:1)
B: Maus (Mus musculus; SEQ ID NO:2)
C: Mensch (SEQ ID NO:3)
D: Huhn (Gallus gallus; SEQ ID NO:4)

Fig. 6: Alignment der in Fig. 5 gezeigten Sequenzen.

Fig. 7: zeigt die bevorzugte, aus Ratte (Rattus norvegicus) stammenden α

| -SMA Sequenzen der vorliegenden Erfindung | (SEQ ID NO:) |
|---|---|
| A: 5'Genbereich des α-SMA-Gens: -698 bis +18 | (5) |
| B: 5'Genbereich des α-SMA-Gens: -124 bis +18 | (6) |
| C: 5'Genbereich des α-SMA-Gens: -189 bis +18 | (7) |
| D: 5'Genbereich des α-SMA-Gens: -214 bis +18 | (8) |
| E: 5'Genbereich des α-SMA-Gens: -244 bis +18 | (9) |
| F: 5'Genbereich des α-SMA-Gens: -484 bis +18 | (10) |
| G: 5'Genbereich des α-SMA-Gens: -521 bis +18 | (11) |
| H: 5'Genbereich des α-SMA-Gens: -189 bis -125 | (12) |
| I: 5'Genbereich des α-SMA-Gens: -214 bis -190 | (13) |
| J: 5'Genbereich des α-SMA-Gens: -244 bis -215 | (14) |
| K: 5'Genbereich des α-SMA-Gens: -484 bis -245 | (15) |
| L: 5'Genbereich des α-SMA-Gens: -521 bis -485 | (16) |
| M: 5'Genbereich des α-SMA-Gens: -698 bis -522 | (17) |

Fig. 8A: β-1-Globin-Gen von Oryctolagus cuniculus; Intron II /Exon III-Übergang; Acc.No: V00882: Sequenz 1250-1343 (SEQ ID NO:30)

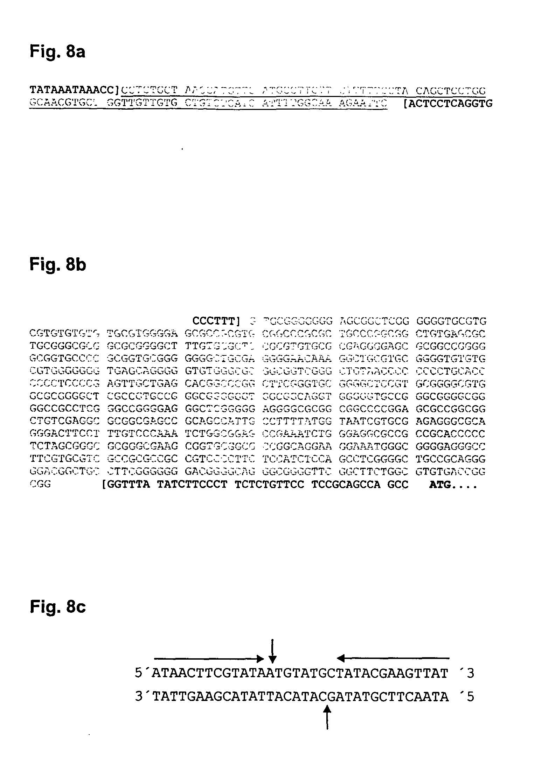

Fig. 8B: Cytoplasmisches β-Actin-Gen von Gallus gallus; Acc. No: X00182, Intron I (544-1542); gezeigt ist die Sequenz von 550 bis 1503 (SEQ ID NO:31).

Fig. 8C: Skizze einer floxP-site: Die FloxP-Sequenz (siehe auch SEQ ID NO:33) besteht aus 13 bp invertierten Repeats (horizontale Pfeile), die eine 8 bp asymmetrische Core-Region flankieren, welche an zwei Stellen (vertikale Pfeile) durch Cre geschnitten wird.

### Detaillierte Beschreibung der Erfindung

Die vorliegende Erfindung beruht auf den überraschenden Befund, dass durch spezifische regulatorische Sequenzen des α-SMA-Gens, insbesondere solche die eine oder mehrere der in Fig. A bis M von Fig. 7 (SEQ ID NOs:5 bis 17) gezeigten Sequenzen enthalten, die Genexpression der hiermit operativ verknüpften funktionellen Nukleinsäuresequenz so z. B. eines Reportergens oder eines anderen funktionellen Gens in Myofibroblasten oder myofibroblastähnlichen Zellen induziert wird.

Die Nukleinsäuresequenz der Ausführungsform (1) der Erfindung enthält einen oder mehrere funktionelle Bereiche aus dem regulierenden Sequenzbereich des α-SMA-Gens. Dieser regulierende Sequenzbereich kann dabei von Säugern, insbesondere von Primaten oder Nagern oder Vögeln und besonders bevorzugt von Ratte, Maus, Mensch oder Huhn stammen.

In der bevorzugten Ausführungsform (2) stammt der regulierende Sequenzbereich, wie vorstehend erwähnt, von der Ratte. Besonders bevorzugt ist dabei, dass der funktionelle Bereich eine transkriptionsaktivierende Nukleinsäuresequenz aufweist und besonders bevorzugt aus der in Fig. 7C gezeigten Sequenz stammt. Hierbei sind insbesondere solche transkriptionsaktivierende Sequenzen bevorzugt, die wenigstens 10, vorzugsweise wenigstens 40 und besonders bevorzugt wenigstens 60 konsekutive Basen aus der in Fig. 7C gezeigten Sequenz aufweisen und besonders bevorzugt die in Fig. 7B oder 7C gezeigten Sequenzen (SEQ ID NOs:6 oder 7) umfassen. Alternativ oder zusätzlich hierzu kann der funktionelle Bereich eine zelltypspezifisch oder differenzierungsabhängig regulierende Nukleinsäuresequenz sein, bevorzugt eine solche, die aus der Sequenz -698 bis -190 der Fig. 7A (Nukleotide 1 bis 509 von SEQ ID NO:5), besonders bevorzugt aus der Sequenz -698 bis -215 der Fig. 7A, stammt. Dabei sind solche zelltypspezifische Sequenzen bevorzugt, die wenigstens 25, vorzugsweise wenigstens 35, konsekutive Basen aus der Sequenz -698 bis -190 der Fig. 7A aufweisen und besonders bevorzugt die in Fig. 7I oder 7M gezeigten Sequenzen umfassen.

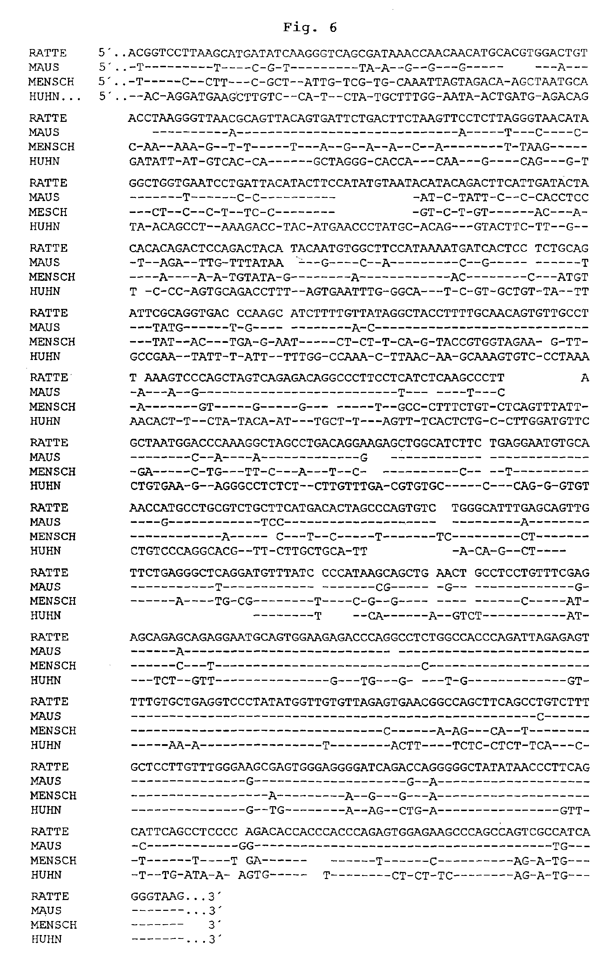

Gemäß der bevorzugten Ausführungsformen (5) - (10) stammt der regulierende Sequenzbereich alternativ von der Maus, dem Menschen oder dem Huhn, wobei die funktionellen Bereich vorstehend unter (5) bis (10) definiert sind. Besonders bevorzugt im Sinne der vorliegenden Erfindung ist auch hier, dass die transkriptionsaktivierende Nukleinsäuresequenz wenigstens 10, bevorzugt wenigstens 40 und besonders bevorzugt wenigstens 60 konsekutive Basen aus der unter (6), (8) oder (10) definierten transkriptionsaktivierenden Sequenz aufweist bzw. wenigstens 25, vorzugsweise wenigstens 35, konsekutive Basen aus der vorstehend unter (6), (8) oder (10) definierten zelltypspezifischen Sequenz aufweist. Insbesondere sind solche Teilsequenzen aus in den SEQ ID NOs:18, 19 und 20 gezeigten Sequenzen bevorzugt, die den Sequenzen 7A - 7M der Sequenz aus Ratte entsprechen (gemäss dem Alignment in Fig. 6).

Die weitere funktionelle Nukleinsäuresequenz gemäß Ausführungsform (1) der Erfindung kann dabei eine peptid- oder proteinkodierende DNA-Sequenz (einschließlich jedoch nicht eingeschränkt auf Reportergene, Sequenzen, die für pharmakologisch aktive Proteine kodieren und regulatorische DNA-Sequenzen) oder eine funktionelle RNA-kodierende Sequenz (einschließlich jedoch nicht eingeschränkt auf ein Ribozym) sein.

Geeignete Reportergene sind z. B. die in der nachfolgenden Tabelle 1 aufgeführten Gene. Andere funktionelle Gene im Sinne der vorliegenden Erfindung sind z. B. Gene, die für pharmakologisch aktive Proteine oder Peptide kodieren.

**Tab. 1:**

| Beispiele möglicher Reporter, deren Expression durch die α-SMA-5'-Sequenz kontrolliert werden kann | | | |
|---|---|---|---|
| Reporter | Detektion | Sequenz (SEQ ID NO) | Referenz |
| destabilisiertes EGFP | 488-507 nm | 21 | EP-A-0892047 |
| red fluorescent Protein | 538-583 nm | 22 | |
| GFP und Varianten | | | |
| z.B. Blue Variant | 380-440 nm | 23 | EP-A-0892047 |
| Renilla Luziferase | | 24 | |
| Luziferase Firefly | Lumineszenz | 27 | |
| SEAP¹ | BCIP², NBT³ | 26 | |
| CAT⁴ | | 28 | |
| hGH⁵ | | 29 | JP 1985234584 |
| β-Galactosidase | x-Gal⁷ | 25 | |
| Horseradish Peroxidase | DAB⁶, AEC⁷ | | EP-A-0234075 |

| | | | |
|---|---|---|---|
| ¹ SEAP: secreted alkaline phosphatase | | | |
| ² BCIP: 5-Bromo-4-chloro-3-Indolylphosphat | | | |
| ³ NBT: Nitroblue-Tetrazolium | | | |
| ⁴ CAT: Chloramphenicol-Acetyltransferase | | | |
| ⁵ hGH: human growth hormone | | | |
| ⁶ DAB: 3,3'-Diaminobenzidin | | | |
| ⁷ AEC: 3-Amino-9-Ethylcarbazol | | | |

Die eukaryontischen Zellen oder Organismen gemäß Ausführungsform (15) der vorliegenden Erfindung können durch dem Fachmann wohlbekannte Transfektions-, Transformations- oder Infektionsverfahren von Ausgangszellen bzw. Infizieren von ES-Zellen in Blastozysten der Organismen und Einsetzen in die Leihmutter erhalten werden.

Bislang war es nicht möglich die myofibroblastische Aktivierung durch einen Reporter zu sichten, da ein regulierender Sequenzbereich, der spezifisch auf den Aktivierungsübergang von ruhenden Zellen zu myofibroblastischen Zellen anspricht, nicht gefunden wurde. Durch die Fusion des Sequenzbereichs von -698 bis +18 (SEQ ID NO:5) mit einem Reportergen (siehe Tabelle 1) und Insertion in einen Transfervektor und anschließender Transfektion kann die myofibroblastische Aktivierung gesichtet werden (Fig. 1). In bisherigen Methoden wurde für eine Sichtung des myofibroblastischen Aktivierungsvorgangs immunzytologische oder immunhistologische Verfahren gewählt, die mehrere Arbeitschritte wie Fixieren der Zellen, Antigenblocken, Antikörperinkubation und Detektion beinhalteten (F. Hofman, Current protocols in immunology, Chapt. 5.8: Immunohistochemistry; Eds. J.E. Coligan et al., National Institute of Health, USA (1996)). Zur immunzytologischen bzw. immunhistologischen Detektion von Myofibroblasten konzentrierten sich die Verfahren auf die immunochemische Anfärbung des α-SMA-Proteins. Quantitative Aussagen über die Aktivierung z.B. durch ein toxikologisches Reagenz waren allerdings auf diese Weise nicht möglich. In der vorgelegten Erfindung wurde als α-SMA-5'-Sequenz kontrollierter Reporter des myofibroblastischen Transformationsprozeß vorzugsweise lumineszierende Reporterproteine gewählt und die Aktivierung der Zellen quantitativ und sehr empfindlich durch ein Luminometer (MLX Microplate Luminometer der Fa. Dynex Technologies, Chantilly, USA) gemessen (Fig. 1). Durch eine quantitative Reporterbestimmung ergibt sich durch das Verfahren erstmals die Möglichkeit toxikologische und pharmakologische Studien quantitativ durchzuführen, die dann Aussagen über das myofibroblastische Aktivierungspotential der Substanz ergeben und damit die Möglichkeit der Detektion des chronisch relevanten Organschädigungspotential.

Die Bestimmung des myofibroblastischen Übergangs an Zellen, die den Vektor mit dem α-SMA-5-Sequenz-Reporterfusionkonstrukt beinhalten, birgt auch die Möglichkeit, therapeutische Medikamentsichtungen durchzuführen; hier kann eine Verminderung der myofibroblastischen Aktivierung, z.B. induziert durch ein Toxin oder einen fibrogenen Wachtumsfaktor wie z.B. Transforming Growth Factor beta (TGF β), anhand des α-SMA-5'-Sequenz kontrollierten Reporters ausgelesen werden.

Das vorgelegte Verfahren, in dem a-SMA-5'-Sequenzbereiche (-698 bis +18) zur Reportersteuerung genutzt werden, umfaßt neben der Sichtung der myofibroblastischen Aktivierung die Isolierung von bestimmten Zellen anhand der α-SMA-5'- gesteuerten Reporterexpression. Dies wird in dem vorgestellten Verfahren vorzugsweise durch operative Verknüpfung von α-SMA-5'-Sequenzbereichen mit einem fluoreszierenden Reporter (z.B. GFP siehe Tabelle 1) durchgeführt, so daß nach Einbringen des Vektorkonstruktes in einen Organismus oder einer Zellpopulation die Isolierung von myofibroblastisch aktivierten oder SM-differenzierten (SM: smooth muscle) Zellen aus einem Zellgemisch oder Verbandes aufgrund der Fluoreszenz mithilfe eines FACS-Gerätes (FACS: Fluorescence activated cell sorting) durchgeführt werden kann. Alternativ erlaubt die Verknüpfung des α-SMA-5'- Sequenzbereichs mit anderen Reportern die Isolierung von aktivierten oder SMA-exprimierenden Zellen durch Mikrodissektion unter licht- oder fluoreszenzmikroskopischer Kontrolle.

Die vorgelegte Erfindung umfaßt ebenfalls die Rolle des Sequenzbereichs -698 bis +18 des α-SMA-Gens für die spezifische Genexpression in embryonalen Stammzellen (ES), die in Embryoid Bodies zu embryonalen Kardiomyozyten, glatten Muskelzellen oder Skelettmuskelzellen differenzieren können (Evans, M. J., Kaufmann, M. H., Nature, 292, 154 - 156 (1981)). Der Vergleich verschiedener stabil in ES-transfezierter Konstrukte mit unterschiedlichen Domänen des α-SMA-5'- gelegenen Sequenzbereichs zeigte, daß für eine Selektion von Kardiomyozyten, Skelettmuskelzellen und Glatten Muskelzellen aus embryonalen Zellaggregaten eine Reportersteuerung durch den Sequenzbereich -189 bis +18 (Fig. 7C; SMA-190) nicht ausreicht, sondern zusätzliche 5-gelegene Domänen im Bereich -698 bis -190 des α-SMA-Gens ( Seq. I, J, K, L und M der Fig. 7) notwendig sind (Fig. 2 ).

Die Erfindung beinhaltet ebenfalls die operative Verknüpfung des α-SMA-5'- Sequenzbereichs oder Teilbereiche der Sequenzen (Seq. A bis M von Fig. 7) mit anderen die Expression beeinflußenden Nukleinsäuresequenzen. Beispiel sind Intronsequenzen wie die des β-Globin- (siehe Fig. 8a, SEQ ID NO:30) oder des Huhn-β-Aktin-Gens (siehe Fig. 8b, SEQ ID NO:31), aber auch andere Enhancersequenzen wie die des viralen CMV wie in dem Vektor von Fig. 3. Aber auch andere hier nicht genannte expressionsbeeinflußende Sequenzen können mit den α-SMA-5'- Sequenzen oder Domänen aus denen (Seq. A bis M von Fig. 7) operativ verknüpft werden.

Wird nicht ein Reportergen, sondern ein anderer peptid- oder proteinkodierender Genbereich mit den α-SMA-5'- Sequenzbereichen (SEQ ID NOs:5 bis 17) verknüpft, kommt es zu einer α-SMA-5'-gesteuerten Expression des operativ verknüpften Genbereichs. In der bevorzugten Ausführungsform der Erfindung wird Sequenz A von Fig. 7 für eine Steuerung der Expression des Genbereichs von Interesse benutzt. Fig. 1 oder Fig. 3 zeigt die differenzierungsabhängige Expression unter transkriptioneller Kontrolle der Sequenz A von Fig. 7: Die transkriptionelle Kontrolle durch Sequenz A verhindert die Expression in Myoblasten oder ruhenden myofibroblastischen Vorläufer, während sie nach Myotubendifferenzierung oder nach myofibroblastischer Transdifferenzierung zur Expression des verknüpften Genbereichs führt. Wird Sequenz C von Fig. 7 mit dem Zielgen verknüpft, kommt es auch zur Expression in vielen Zelltypen wie z.B. in Fibroblasten, bei Verknüpfung der Sequenzen A, F, G von Fig. 7 in myofibroblastischen Zellen oder SMC-Abkömmlingen. Der durch α-SMA-5'- Sequenzbereiche (Seq. A bis M von Fig. 7) transkriptionell gesteuerte Genbereich kann wiederum ebenfalls mit zusätzlichen Sequenzdomänen verknüpft werden. Die Verknüpfung unter Berücksichtigung des Leserasters mit kodierenden Sequenzen für bestimmte Tags oder Reporterdomänen (Fig. 3b) ermöglicht die Verfolgung der Genexpression oder die Aufreinigung Proteins oder Peptids des durch α-SMA-Sequenzen transkriptionell gesteuerten Genbereichs.

Durch die operative Verknüpfung der α-SMA-5'-Sequenzbereiche (Seq. A bis M von Fig. 7) mit Sequenzen, die RNA kodierende DNA oder Genbereiche für Signalmediatoren enthalten, kann funktionell in den Phänotyp myofibroblastischer Zellen eingegriffen werden. Nach Insertion solcher Konstrukte in einen Vektor, der sich zur Gentherapie eignet (wie z.B. verschiedene Generationen von adenoviralen, retroviralen oder lentiviralen Vektoren und deren Abkömmlinge (Ory, D. S. et al., Proc. Natl. Acad. Sci. 93: 11400 - 11406 (1996); Feng, M. et al., Nat. Biotechnol. 15: 866 - 870 (1997)) bzw. nackte oder in Aggregaten mit geladenen Polymeren oder Liposomen zusammengefaßte Plasmidvektoren) (Hengge, U. et al., Nat. Genet. 10: 161-166 (1995); Gottschalk, S. et al., Gene Ther. 3: 448 - 457 (1996)) wird die Voraussetzung für gentherapeutische Ansätze myofibroblastischer Zellen, myofibroblastähnlicher, oder SM-ähnlicher Zellen geschaffen, die bei sklerosierenden Erkrankungen und Tumorerkrankungen genutzt werden können.

Die operative Verknüpfung von Sequenzbereichen aus Sequenz A bis M aus Fig. 7, wobei in dieser Erfindung vorzugsweise die Sequenz A aus Fig. 7 benutzt wurde, mit dem Enzym für die zyklische Rekombination (Cre) (SEQ ID NO:32) ermöglicht es, in myofibroblastisch-differenzierten Zellen Genbereiche, die mit einer floxP-Sequenz (Fig. 8c, SEQ ID NO:33) flankiert wurden, neu zu rekombinieren. Hier kann nach Funktion des Cre-Enzyms und der Orientierung der floxP-Sequenzen eine Inversion, eine Excision, Integration oder Translokation des gefloxten Genbereichs unter Kontrolle der gewählten regulierenden α-SMA-5'- Sequenzbereiche erfolgen. In dieser Erfindung gewählte mögliche Konstruktionen des Vektors sind in Fig. 4 dargestellt.

In dem Verfahren (17) gemäß der vorliegenden Erfindung werden transfizierte ES-Zellen in Blastozysten eines Wirtsorganismus injiziert und in die Leihmutter eingesetzt (V. Papaioannou, R. Johnson, Gene Targeting. A Practical Approach, ed. A.L: Joyner, Oxford UK (1993); T. Doetschman, Transgenis Animal Technology. A Laboratory Handbook, Academic Press Inc. San Diego, USA (1994)). Nach Rückkreuzungen der geborenen und herangewachsenen transgenen Chimäre werden homozygote Tiere intoxiziert und auf ihre transgene Genexpression untersucht.
Alternativ können in dem Verfahren (17) die Konstrukte ohne Vektorrückrad durch Miokroinjektion in den Vorkern eingebracht werden und zu transgenen Organismen führen (J.W. Gordon et al., Methods Enzymol. 101: 411-33 (1983); B. Hogan et al., Manipulating the mouse embryo. A laboratory manual, Gold Spring Harbor, NY (1994)).

Organismen gemäß der Ausführungsformen (15) und (17) der vorliegenden Erfindung umfasst Säuger einschließlich, aber nicht eingeschränkt auf Primaten, Nager und Huftiere (wobei es sich - falls humane Organismen unter den betreffenden nationalen oder regionalen Patentvoraussetzungen nicht schutzfähig sind - bei den Säugern um nichthumane Säuger handelt). Die eukaryotischen Zellen gemäß Ausführungsform (15) umfassen alle möglichen Zellen und Zelllinien aus den obengenannten Säugern.

Die Arzneimittel gemäß Ausführungsform (20) der vorliegenden Erfindung können - in Abhängigkeit von der Applikationsform - neben den erfindungsgemäßen Nukleinsäuresequenzen/Vektoren noch handelsübliche Zusatzstoffe, Verdünnungsmittel und dergleichen enthalten.

Die Erfindung wird anhand der folgenden, nicht einschränkenden Beispiele näher erläutert.

### Beispiele

### Materialien Und Methoden

**Tabelle 2:**

| Verwendete Primer | | |
|---|---|---|
| Primer | Sequenz (SEQ. ID. NO) | Bereich im α-SMA-Gen |
| SMA-710F | 5'-GTC CTT AAG CTT GAT ATC AAG GGT CAG CG (37) | - 710 bis -682 |
| SMA-30R | 5'-GGC TTC TGA ATT CTG GGT GGG TGG TGT CT (38) | +1 bis +29 |
| SMA-245F | 5'-TGT TCT CAG GCT CAG GAT G (39) | -252 bis -235 |
| SMA-190F | 5'-CTG TTT CGA GAG AGC AAA GCT TAG G (40) | -203 bis -181 |
| SMA-100 | 5'-GAG TGA ACG GCC AGC TTC (41) | um -100 |

Folgende kommerziell erhältliche Vektoren wurden im Verlauf der Arbeit benutzt:

Als allgemeine Klonierungsvektoren dienten pBluescript-SKII+ (Clontech, Heidelberg) und pGEM3Z (Promega, Mannheim). Bereiche des α-SMA-Promoter wurden anschließend in den promoterlosen Luciferase-Vektor pRL-null (Promega, Mannheim) kloniert. Um die Aktivität erzeugter Konstukte zu vergleichen wurden aus der pRL-Familie zusätzlich pRL-SV40 und pRL-CMV oder auch Vektoren der pGL3-Familie (Promega, Heidelberg) verwendet.

### Beispiel 1: Herstellen der α-SMA-5'-Sequenz

Aus isolierter Ratten-DNA wurde zunächst ein 736bp langes Fragment aus dem 5'-Promoterbereich des α-SMA-Gens amplifiziert und kloniert. Dazu erfolgte zunächst eine PCR-Amplifikation des Bereichs von -708 bis +28 aus genomischer Ratten-DNA mit den Primer SMA-710F (HindIII) und SMA-30R (EcoRI), die die angegebenen Restriktionsschnittstellen in ihrer Sequenz beinhalten. Nach Restriktion wurde das Fragment (-698 bis +18) in pRL-null durch Ligation inseriert und der resultierende Vektor pRL-SMA-700 für Untersuchungen an eukaryontischen Zellen in *E. coli* K12-Stämmen amplifiziert (siehe auch Sequenz von Fig. 7A).

### Beispiel 2: Sichtung der myofibroblastischen Differenzierung durch Reporterexpression unter Kontrolle des 5'- Bereichs des α- SMA-Gens

A. Herstellen von durch α-SMA-5'-Sequenzbereiche regulierten Reportervektoren
   Für die Herstellung der α-SMA-5-Genbereich-Reporterkonstrukte wurden in Reportervektoren unterschiedliche 5'-Genbereichen des α-SMA inseriert. Das mit den Primern SMA-710-F und SMA-30-R hergestellte Amplifikat (s. o.) von genomischer Ratten-DNA wurde mit EcoRI und HindIII geschnitten und in pBSSK + (Stratagene) kloniert und so pB-SMA-700 erzeugt. Unter Nutzung der vorhandenen Restriktionschnittstellen im 5'-Bereich des α-SMA-Gens wurden die Fragmente SMA-480 (PstI), SMA-215 (PvuII) und SMA-125 (DraII) erzeugt, indem sie mit EcoRI und dem spezifischen Restriktionsenzym aus pB-SMA-700 geschnitten wurden und zunächst in pGEM (Promega) oder pB-SK (Stratagene) kloniert wurden. Dabei entstanden folgende Plasmide: pGEM-SMA-480, pGEM-SMA-215 und pB-SMA-125. Mit den Primer SMA-245F (PstI) oder SMA-190F(HindIII) als sense Primer und SMA-30R als antisense Primer wurden spezifische Fragmente generiert, die anschließend über ihre primer-assoziierten Restriktionsschnittstellen kloniert wurden, dadurch entstanden pGEM-SMA-245 und pB-SMA 190.
   Alle Promoterfragmente des α-SMA-Promoter wurden über EcoRI/HindIII oder EcoRI/SacI in pRL-null kloniert, wodurch 6 unterschiedliche lange Deletionsreporterkonstukte SMA-700 bis SMA-125 erzeugt wurden.
B. Transiente Transfektion: Transfektionen wurden mit dem Transfektionsreagenz FuGENE™6 (Roche Molecular Biochemicals, Mannheim) durchgeführt. Pro Ansatz wurde 1,5 µg Plasmid-DNA verwendet, die sich aus 1,2 µg pRL-700 und 0,3µg pGL3-Kontrollplasmid zusammensetzten, was einem DNA/Reagenz-Verhältnis von 1 : 3 entspricht. Für die Tranfektionen wurden ruhende Hepatische Sternzellen (HSC), die durch enzymatische Leberperfusion und Dichtegradientenzentrifugation aus der Rattenleber isoliert wurden (Knock, D. S., de Leeuw, A. M., Exp. Cell. Res., 139, 468 471 (1982)) und nach Kultur in Myofibroblasten differenzierte HSC verwendet (Geerts, A. et al., J. Hepatology, 9, 59 - 68 (1989)).
C. Bestimmung der Reporteraktivität unter Kontrolle von Sequenz A in ruhenden HSC und in myofibroblastischen HSC: Eine Messung der Aktivitäten erfolgte mit dem Dualen-Luciferase Reporter-Assay-System (DLR™, Promega, Mannheim). In den transienten Transfektionen zeigten nur myofibroblastische Zellen eine deutliche Aktivität des pRL-700-Reporterkonstrukt. α-SMA-negative Zellen zeigen Reporteraktivitäten, die unterhalb von einem Prozent der relativen Aktivität lagen. In Myofibroblasten wurde der Reporter um den Faktor 14 induziert. Ruhende Zellen hatten keine Aktivitäten (>1%) (Fig. 1). Damit kann das Konstrukt nach Transfektion zur Sichtung der myofibroblastischen Aktivierung verwendet werden.

### Beispiel 3: Reporter unter Kontrolle einer α-SMA 5'-Sequenz in stabil transfizierten Organismen und Zellen

A. Konstruktion eines Reporter-Vektors unter Kontrolle des α-SMA: Aus einem modifizierten GFP-Expressionvektor (pCAGGS) (Ikawa, M. et al., Develop. Growth Differ. 37: 455 - 459 (1995)) mit einem Fragment des Chicken-β-Actin-Promoter und dem ersten Intron des Chicken-β-Actins (SEQ ID NO:31) wurde das kardiale Promoterelement durch einen Restriktionsverdau mit SnaBI und ApaI exzidiert und mit den jeweiligen α-SMA-5'-Sequenzbereichen (SEQ ID NO:5 oder SEQ ID NO:7) nach Verdau von pRL-700 oder pRL-190 mit XhoI und EcoRI ersetzt. Nach Aufreinigung der Banden über ein Agarosegel wurden die Enden aufgefüllt, um eine 'Blunt End'-Klonierung durchführen zu können. Um eine Religation des Vektors zu verhindern, wurden bei diesem zusätzlich eine Dephosphorylierung der 5'-Enden durchgeführt. Nach einer Ligationsreaktion und Transformation in *E. coli* wurden positive Klone isoliert, deren Plasmid-DNA aufgereinigt und sequenziert und so der Einbau der α-SMA-5'-Sequenzbereiche in der richtigen Orientierung überprüft. Die Sequenzierung wurde unter Verwendung des markierten Primers SMA-100 (TM=63°C), der im Bereich -100 des α-SMA-Promoter bindet, durchgeführt. Die Konstrukte sind in Fig. 2b dargestellt.
B. Sichtung und Isolierung von stabilen Transfektanten anhand der α-SMA-5-Sequenz (Sequenzen von Fig. 7A und 7C) kontrollierten Reporteraktivität: Zur Erzeugung von stabilen ES-Transfektanten wurden die beiden GFP-Reporterkonstrukte pSMA-GFP-700 und -190 (Abb.2b) hergestellt und in embryonale murine D3-Stammzellen transfiziert. Die stabil transfizierten Klone wurden mittels ihrer Neomycin (G418)-Resistenz, die im GFP-Konstrukt beinhaltet ist, selektioniert. Dazu wurde 24 Stunden nach der Transfektion zum erstenmal 50 µg/ml Neomycin (10 mg/ml, GibcoBRL, Eggenstein) zugesetzt.

Zur Differenzierung von Stammzellen wurden diese ohne Feederzellen und ohne Zugabe von LIF in DMEM (440 mg D-Glukose, 110 mg Natriumpyruvat, L-Glutamin, Sigma) mit 20 % FCS (GibcoBRL, Gaithersburg, MD), 1x nicht-essentiellen Aminsäuren (GibcoBRL), 1x Penicillin-Streptomycin (GibcoBRL) und 0,1 mM β-Mercaptoethanol (Sigma) kultiviert. Für den Ansatz von Embryoid Bodies (EB) wurden 2,25 x 10⁵ bzw. 4,25 x 10⁵ Zellen in 1 ml Kulturmedium verdünnt. Jeweils 20 µl der Zellsuspension (450 oder 800 Zellen) wurden als hängende Tropfen kultiviert (Drab, M. et al., FASEB, 11: 905-911 (1997) und Kolossov, E. et al., J. Cell. Biol., 143: 2045-2056 (1998)).

Die Immunhistolgie an EB, die mit dem pSMA-GFP-190 Promoterfragment transfiziert wurden, zeigte, daß GFP-positive Zellareale nicht immer eine α -SMA-Expression hatten und nicht alle α-SMA-positiven Zellen GFP exprimierten. GFP-positive Zellen konnten Actinin-exprimierenden kardialen oder skelettalen Muskelzelltypen zugeordnet werden. Caldesmon-positive glatte Muskelzellen wiesen keine GFP-Expression auf.

Im Gegensatz dazu zeigte das pSMA-GFP-700 Konstrukt eine spezifische GFP-Expression, die GFP-Expression konnte dabei sowohl in kardialen und skelettalen Bereichen als auch in caldesmonpositiven glatten Muskelzellen gefunden werden. Das gesichtete Reportermuster unter Kontrolle der Sequenz A in Fig. 5 des α-SMA-Gens entspricht in differenzierten ES-Zellen demjenigen während der Embryogenese.

Anhand der Reporteraktivität konnten Zellen durch Trypsinierung vereinzelt werden (Fig. 2.a) und getrennt untersucht werden.

### Beispiel 4: Herstellen von Mäusen mit transgener Genexpression in myofibroblastischen Zellen

A. Stabile Transfektion des in Fig. 2b/3 gezeigten Konstrukts in ES-Zellen: Zur Herstellung von stabil transfizierten embryonalen Stammzellen wurden die α-SMA-Transgen-Vektoren zunächst durch einen Restriktionsverdau, dessen Schnittstelle im Ampicillingen liegt, linearisiert und nach einer Aufreinigung in H₂O gelöst (1 µg/µl). Anschließend wurde eine Elektroporation vorgenommen. Dazu wurden 5 x 10⁶ ES-Zellen in 0,7 ml PBS aufgenommen, resuspendiert und in eine Küvette überführt. Pro Elektroporationen wurden 30 µg linearisierte Plasmid-DNA in einem Volumen von 100 µl verwendet. Nach Zugabe der DNA in die Küvette erfolgte zunächst eine Inkubation für 10 min auf Eis, die der Bildung von Präkomplexen diente. Danach wurde mit einem Elektroporator der Firma Biorad (München) bei 500 µF und 0,24 V ein Impuls erzeugt. Zur Regeneration wurden die Zellen zunächst für 20 Minuten auf Eis inkubiert und anschließend auf neomycinresistene Feederzellen plattiert.
   Die ES- Zellen wurden in Blastozysten einer Wirtsmaus injeziert und in die Leihmutter eingesetzt (V. Papaioannou, R. Johnson, in Gene Targeting. A Practical Approach, ed. A.L: Joyner, Oxford UK (1993); T. Doetschman, Transgenis Animal Technology. A Laboratory Handbook, Academic Press In.c San Diego, USA (1994)). Nach Rückkreuzungen der geborenen und herangewachsenen transgenen Chimäre wurden homozygote Tiere intoxiziert und auf ihre transgene Genexpression untersucht.
   Alternativ können Konstrukte ohne Vektorrückrad (siehe Fig.2b) durch Miokroinjektion in den Vorkern eingebracht werden und zu transgenen Mäusen führen (J.W. Gordon et al., Methods Enzymol. 101: 411-33 (1983); B. Hogan et al., in Manipulating the mouse embryo. A laboratory manual, Gold Spring Harbor, NY (1994)).
B: Chronische Leberschädigung durch Intoxikation und myofibroblastische Sichtung
   Zur Induktion einer chronischen Leberschädigung wurden zwei wöchentliche Gaben von 4 % igem Tetrachlorkohlenstoff in Mineralöl (6 ml/kg Körpergewicht) den Mäusen intraperitoneal 2, 4, 6 oder 8 Wochen lang verabreicht. Da Tetrachlorkohlenstoff vor allem zu einer Hepatitis führt, wurde in den Lebern transgener GFP-Mäuse die Aktivierung von myofibroblastischen Zellen gesichtet. Daneben wurden die Mäuse auf eine Pulmonitis und Nephritis sowie auf das Auftreten myofibroblastischer Zellen in der Lunge und der Niere untersucht.

## Patentansprüche

1. Nukleinsäuresequenz, umfassend
(i) einen oder mehrere funktionelle Bereiche aus dem regulierenden Sequenzbereich des alpha-Smooth-Muscle-Actin-Gens (α-SMA-Gens) und
(ii) wenigstens eine weitere funktionelle Nukleinsäuresequenz, die mit der Sequenz (i) operativ verknüpft ist.

2. Nukleinsäuresequenz nach Anspruch 1, wobei der regulierende Sequenzbereich des α-SMA-Gens von Säugern, insbesondere von Primaten oder Nagern, oder Vögeln stammt und besonders bevorzugt von Ratte, Maus, Mensch oder Huhn stammt.

3. Nukleinsäuresequenz nach Anspruch 2, wobei der regulierende Sequenzbereich von der Ratte stammt und die Nukleinsäuresequenz vorzugsweise einen oder mehrere funktionelle Bereiche aus der in Fig. 5A gezeigten Sequenz (SEQ ID NO:1), insbesondere aus der in Fig. 7A gezeigten Sequenz (SEQ ID NO:5), umfasst.

4. Nukleinsäuresequenz nach Anspruch 3, wobei der funktionelle Bereich
(i) eine transkriptionsaktivierende Nukleinsäuresequenz ist und insbesondere aus der in Fig. 7C gezeigten Sequenz (SEQ ID NO:7) stammt, und wobei vorzugsweise die transkriptionsaktivierende Sequenz wenigstens 10, bevorzugt wenigstens 40 und besonders bevorzugt wenigstens 60 konsekutive Basen aus der in Fig. 7C gezeigten Sequenz aufweist und besonders bevorzugt die in Fig. 7B oder 7C gezeigte Sequenz (SEQ ID NOs:6 oder 7) umfasst; oder
(ii) eine zelltypspezifische Nukleinsäuresequenz ist und insbesondere aus der Sequenz -698 bis -190 der Fig. 7A (Nukleotide 1 bis 509 von SEQ ID NO:5), besonders bevorzugt aus der Sequenz -698 bis -215 der Fig. 7A (Nukleotide 1 bis 484 von SEQ ID NO:5) oder aus der Sequenz der Fig. 7I (SEQ ID NO:13) stammt, und wobei vorzugsweise die zellspezifische Sequenz wenigstens 25, bevorzugt wenigstens 35 konsekutive Basen aus der Sequenz -698 bis -190 der Fig. 7A aufweist und besonders bevorzugt die in Fig. 7I oder 7M (SEQ ID NO:13 oder 17) gezeigte Sequenz umfasst.

5. Nukleinsäuresequenz nach Anspruch 3 oder 4, wobei die Nukleinsäuresequenz sowohl eine transkriptionsaktivierende als auch eine zelltypspezifische Nukleinsäuresequenz umfasst und/oder eine der in SEQ ID NOs:5 bis 17 gezeigten Sequenzen aufweist.

6. Nukleinsäuresequenz nach Anspruch 2, wobei der regulierende Sequenzbereich von der Maus stammt und die Nukleinsäuresequenz vorzugsweise einen oder mehrere funktionelle Bereiche der in Fig. 5B gezeigten Sequenz (SEQ ID NO:2) insbesondere aus der in SEQ ID NO:18 gezeigten Sequenz aufweist.

7. Nukleinsäuresequenz nach Anspruch 6, wobei der funktionelle Bereich
(a) eine transkriptionsaktivierende Nukleinsäuresequenz ist und insbesondere aus den Nukleotiden 490 bis 697 der in SEQ ID NO:18 gezeigten Sequenz stammt und/oder
(b) eine zelltypspezifische Nukleinsäuresequenz ist und insbesondere aus den Nukleotiden 1 bis 489 der in SEQ ID NO:18 gezeigten Sequenz stammt.

8. Nukleinsäuresequenz nach Anspruch 2, wobei der regulierende Sequenzbereich vom Menschen stammt und die Nukleinsäuresequenz vorzugsweise einen oder mehrere funktionelle Bereiche der in Fig. 5C gezeigten Sequenz (SEQ ID NO:3) insbesondere aus der in SEQ ID NO:19 gezeigten Sequenz aufweist.

9. Nukleinsäuresequenz nach Anspruch 8, wobei der funktionelle Bereich
(a) eine transkriptionsaktivierende Nukleinsäuresequenz ist und insbesondere aus den Nukleotiden 513 bis 715 der in SEQ ID NO:19 gezeigten Sequenz stammt und/oder
(b) eine zelltypspezifische Nukleinsäuresequenz ist und insbesondere aus den Nukleotiden 1 bis 512 der in SEQ ID NO:19 gezeigten Sequenz stammt.

10. Nukleinsäuresequenz nach Anspruch 2, wobei der regulierende Sequenzbereich vom Huhn stammt und die Nukleinsäuresequenz vorzugsweise einen oder mehrere funktionelle Bereiche der in Fig. 5D gezeigten Sequenz (SEQ ID NO:4) insbesondere aus der in SEQ ID NO:20 gezeigten Sequenz aufweist.

11. Nukleinsäuresequenz nach Anspruch 10, wobei der funktionelle Bereich
(a) eine transkriptionsaktivierende Nukleinsäuresequenz ist und insbesondere aus den Nukleotiden 497 bis 699 der in SEQ ID NO:20 gezeigten Sequenz stammt und/oder
(b) eine zelltypspezifische Nukleinsäuresequenz ist und insbesondere aus den Nukleotiden 1 bis 496 der in SEQ ID NO:20 gezeigten Sequenz stammt.

12. Nukleinsäuresequenz gemäß einem oder mehreren der Ansprüche 1 bis 11, wobei die weitere funktionelle Nukleinsäuresequenz eine peptid- oder proteinkondierende DNA-Sequenz, insbesondere ausgewählt ist aus Reportergenen, Sequenzen, die für pharmakologisch aktive Proteine kodieren, und regulatorische DNA-Sequenzen, oder eine funktionelle RNA-kodierende Sequenz, insbesondere ein Ribozym, ist.

13. Transkriptionsaktivierende Nukleinsäuresequenzen oder zelltypische Nukleinsäuresequenzen, wie in Ansprüchen 4, 7, 9, oder 11 definiert.

14. Vektor, umfassend eine Nukleinsäuresequenz, wie in Anspruch 13 definiert, oder in dem eine Nukleinsäuresequenz, wie in Ansprüchen 1 bis 12 definiert, inseriert ist.

15. Eukaryontische Zellen oder Organismen, die mit einer Nukleinsäuresequenz, wie in Ansprüchen 1 bis 12 definiert, oder einem Vektor, wie in Anspruch 14 definiert, transient oder stabil transfiziert, transformiert oder infiziert sind, und die vorzugsweise
(i) einen Reporter unter Kontrolle der regulatorischen Sequenzen des α-SMA-Gens exprimieren; oder
(ii) ein pharmakologisch aktives Protein, eine regulatorische DNA-Sequenz oder eine funktionelle RNA-Sequenz unter Kontrolle des α-SMA-Gens exprimieren oder aufweisen.

16. Verfahren zur Herstellung der eukaryontischen Zellen, wie in Anspruch 15 definiert, umfassend das Transfizieren, Transformieren oder Infizieren von Ausgangszellen mit den Nukleinsäuresquenzen, wie in Ansprüchen 1 bis 12 definiert, oder mit Vektoren, wie in Anspruch 14 definiert.

17. Verfahren zur Herstellung der Organismen, wie in Anspruch 15 definiert, umfassend das Injizieren von ES-Zellen, die mit den Nukleinsäuresquenzen, wie in Ansprüchen 1 bis 12 definiert, oder mit Vektoren, wie in Anspruch 14 definiert, transfiziert sind, in Blastozysten der Organismen und Einsetzen in die Leihmutter.

18. Verfahren zur Isolierung oder zur Sichtung von glatten Muskelzellen, Myofibroblasten oder myofibroblastähnlichen Zellen aus einem Zellgemisch, einer Zellpopulation, einem Zellaggregat oder einem Organismus, umfassend die Expression eines Reportergens in einer eukaryontischen Zelle oder ein Organismus gemäß Anspruch 15, die/der einen Reporter unter Kontrolle der regulatorischen Sequenzen des α-SMA-Gens exprimiert.

19. Verfahren zur Manipulation der Genexpression und/oder der Zellfunktion von Glatten Muskelzellen oder Myofibroblasten in Organismen oder Organen, umfassend das Einbringen von Nukleinsäuresequenzen, wie in Ansprüchen 1 bis 13 definiert, oder Vektoren, wie in Anspruch 14 definiert, in die Organismen oder Organe,
(i) wobei das Einbringen in die Organismen oder Organe vorzugsweise unter Nutzung transgener oder knock-out /-in Technologie erfolgt, oder
(ii) wobei das Verfahren vorzugsweise eine gentherapeutischer Fusionsvektorapplikation ist.

20. Arzneimittel, enthaltend eine Nukleinsäuresequenz, wie in Ansprüchen 1 bis 12 oder 13 definiert, oder einen Vektor, wie in Anspruch 14 definiert, wobei das Arzneimittel vorzugsweise zur Manipulation der Genexpression und/oder der Zellfunktion von glatten Muskelzellen oder Myofibroblasten in Organismen oder Organen geeignet ist.

21. Verwendung eines funktionellen Bereichs aus dem regulierenden Sequenzbereich des α-SMA-Gens zur zelltyp- und differenzierungsspezifischen Reporterexpression und zur zelltyp- und differenzierungsspezifischen Genveränderung, wobei vorzugsweise der funktionelle Bereich des α -SMA-Gens
(i) eine Nukleinsäuresequenz, wie in Anspruch 13 definiert, ist;
(ii) eine Nukleinsäuresequenz, wie in Ansprüchen 1 bis 12 definiert, ist und/oder
(iii) in einem Vektor, wie in Anspruch 14 definiert, vorliegt.
